# EUROPEAN PATENT SPECIFICATION

(11) **EP 1 004 299 B1**
(45) Date of publication and mention of the grant of the patent: **04.11.2009**
(21) Application number: 98901030.1
(22) Date of filing: 28.01.1998
(51) Int. Cl.: A61K 31/12, A61K 39/00, A61K 39/395, C07K 16/00, C07K 16/42, C12N 5/06, C12N 5/08, A61P 35/00

(54) **VACCINE PRECURSORS, VACCINES FOR TREATING TUMORS**
IMPFSTOFFVORLÄUFER, IMPFSTOFFE ZUR BEHANDLUNG VON KREBS
PRECURSEURS DE VACCIN, VACCINS POUR TRAITER LES TUMEURS

(30) Priority: 29.01.1997 JP 2829597
(43) Date of publication of application: 31.05.2000
(73) Proprietor: Koyama, Shozo, Nagano 390-02 (JP)
(72) Inventor: KOYAMA, Shozo, Matsumoto-shi, Nagano 390-02 (JP); TANAKA, Satoshi, Matsumoto-shi, Nagano 390 (JP)
(74) Representative: Liedl, Christine
(86) International application number: PCT/JP1998/000351
(87) International publication number: WO 1998/032431

(56) References cited:
- WO-A-96/07403
- WO-A-96/07403
- WO-A-97/01633
- JP-A- 9 221 421
- KOYAMA S ET AL: "A NEW SUBSTANCE (YOSHIXOL) WITH AN INTERESTING ANTIBIOTIC MECHANISMFROM WOOD OIL OF JAPANESE TRADITIONAL TREE (KISO-HINOKI), CHAMAECYPARIS OBTUSA" GENERAL PHARMACOLOGY, PERGAMON PRESS, OXFORD, GB, vol. 28, no. 5, 1997, pages 797-804, XP002059404 ISSN: 0306-3623
- KOYAMA S ET AL: "APOPTOSIS-LIKE (POSSIBLE QUANTUM THERMODYNAMIC) CELL DEATH INDUCED BY YOSHIXOL AND WOOD OIL OF CHAMAECYPARIS OBTUSA (KISO-HINOKI) ON HELA CELL" GENERAL PHARMACOLOGY, PERGAMON PRESS, OXFORD, GB, vol. 28, no. 5, 1997, pages 805-811, XP002059405 ISSN: 0306-3623
- KOYAMA S. et al., "Apoptosis-Like (Possible Quantum Thermodynamic) Cell Death Induced by Yoshixol and Wood Oil of Chamaecyparis Obtusa (Kiso-Hinoki) on HeLa Cell", Vol. 28, No. 5, (May 1997), p. 805-811.
- KOYAMA S. et al., "A New Substance (Yoshixol) with an Interesting Antibiotic Mechanism from Wood Oil of Japanese Traditional Tree (Kiso-Hinoki), Chamaecyparis Obtusa", Vol. 28, No. 5, (May 1997), p. 797-804.

## Description

### TECHNICAL FIELD

This invention relates to the use of a vaccine presursor or vaccine according to the claims for the manufacture of an antitumor agent.

Generally, a part of role on an immune system in organism is to usually defense normal functioning by recognizing an inconvenient substance for the organism which is generated to exclude outside to the organism. For example, this immune phenomenon relates to infectious defense of micro-organism, refusal of cell originated from different species, removal of mutational cells and/or damaged tissue. It is clear that this existence of antigen-antibody reaction has rescued lots of human life by vaccine and antitoxin as a preventive method. Utilizing this immunological mechanism, a first step of conventional cellular destruction for extracting antigenic substance from microorganism, infected cells with a microorganism and/or cancer cell to apply to live attenuated vaccine or inactivated vaccine has been used ultrasonic wave, freezing technique and combination with heat treatment. Moreover, it has been performed that addition of adjutants such as A1(OH) raises antibody titer after being inactivated antigen containing liquid, which was isolated by a centrifuged separation so on, by use of heat treatment, formaldehyde, paraformaldehyde or ultraviolet rays. However, application of heat, acid and alkaline treatments, and using of an organic solvent such as alcohol, ether, acetone or chloroform may cause chemical modification of molecular structure of the targeted antigen resulting in not only decrease in antigenicity but limitation of sufficient induction of immune mechanism with complexity and reasonability. Thus, it has been expected that development of antibody and/or vaccine which is capable to discriminate a targeted macromolecule existed in organism with specificity.

Generally, when antigen receptor (antigen acceptor) which exists on the surface of lymphocytes is encountered to an antigen which is " non- self ", the antigen binds with the receptor (antigen recognition) resulting in a serial functioning of an elimination mechanism. This linkage has been well known to be specific. Moreover, it is known that this antigen receptor is one of protein molecules (polypeptides) which is induced by a gene and, that immunological specificity and immunological memory is created by a structure of the polypeptide with diversity. In other words, in order to promote antigen recognition efficiently and smoothly, lymphocytes produce and release a lot of antigen receptors to bind to acceptors (non-self). This antigen receptor which is released from the cell is called as an antibody. The many trials which utilize these antibodies artificially is one of central subjects in biological and medical research during this century and, many expected results have been provided. However, a diversity of antibody is astronomical. There are many problems to manipulate a targeted antibody artificially. Thus, it has been anticipated to be presented a demonstration with a new scientific concept.

Generally, it has been well known that a property ofB cell is to have immunoglobulin on the surface. Immunoglobulin of the surface on the B cell is membrane-immuno-globulin with transmembrane portion, but not agglutination substance of antibody in blood (secretary immuno-globulin) and, it functions as B cell receptor to recognize a foreign substance (antigen). Activated B cell by that an antigen binds to B cell receptor differentiates to antibody secretary cell (plasma cell). In this case, a property of the produced antibody is to maintain a homologous antigenic specificity with the membrane immunoglobulin which exists on the surface of B cell. A important role ofB cell in an immune system is to produce a specific antibody against an antigen. It is recently understood that diversity of an antibody, which is capable to correspond of numerous antigens with one by one, is generated in a gene level. This recent biological knowledge and technique have been expanded various developments, however, it is also anticipated to propose a method of an antibody production which does not depend on an analysis and determination of newly produced amino acid sequence by gene. In other words, an antibody production due to genetic engineering may spend a lot of time to identify a property of the antigen and to establish a production process. A production cost may become high. It is a reason to be discussed an economic efficiency and/or timely supply of an adequate vaccine when it is needed to use widely as an aim of public health care or prevention.

Immunoglobulin is formed of two components of heavy chain (H chain) and light chain (L chain) which has common amino acid sequence (common fragment; Fc) on the carboxyl terminal of each immunoglobulin, however, amino acid sequence of the amino terminal differs depending on each immunoglobulin. Antigen-recognition site of immunoglobulin is composed from a variable fragment of this H chain and L chain and, a difference of the amino acid sequence induces to produce a difference and diversity of antigen specificity is produced. Thus, although genetic domain cording the common fragment of each H chain and L chain is fixed on one domain, genetic domain cording the variable fragment is divided into some domains (segment). A combination of the segments results in the diversity of antibody. In other words, a paired part between one of H chain and L chain in antibody molecule is a segment which is called Fab, so that two Fab exists in the antibody. A position formed only by two H chains is a segment which is called Fc. Therefore, when an antibody molecule is displayed on a plane, antibody makes a Y shape with two Fab and one Fcl. It has been studied and performed energetically to develop vaccine and/or antibody corresponding to a difference of antigen specificity and structural specificity related to a generation of diversity.

A destruction of a cellular membrane is triggered by Fc segment of the antibody which has been linked with a cell. For this reason, Fc segment is an important segment. A kind of amino acids and sequence from an terminal end to middle part of Fab segment differs corresponding with each antigen. However, amino acids of Fc segment is quite identical even in different kind of antigen. A site which an antibody binds with an antigen (variable fragment) has a different structure corresponding with each antigen. Thus, when an animal is injected with the antibody, a different antibody is produced in an animal corresponding with each antigen. An antigen with a variable fragment which is able to discriminate is called as idiotype antibody. This idiotype antibody has been thought as an ideal antibody which may induce a better specificity of an antibody. Therefore, many researches and developments to find and/produce this kind of antibody have been performed. Diversity of antibody is based on mini-gene system (family) which consists of more than 100 kinds of V (variable) genes, 12 of D (diversity) genes and 4 of J (joining) genes. Also, C (constant = permanent segment) gene is different depending on a function of the antibody and, this segment is not participated in a linkage with an antigen. During a differentiation to an antibody-produced cell, each gene is chosen from each mini-gene family so that V-D-J-C gene is completed by binding with each mini-gene family. The number of related gene are about 4,800 in H chain and about 400 of combination with mini-gene family of V, J and C in L chain. Thus, antigen-linkage segment to form a pair of H chain and L chain become a possibility of diversity with 4,800 times 400 (1,920,000) as a simple calculation. In addition, when gene is rearranged, several DNA codons may inserted into the binding site between V and D or between D and J by a special enzyme so that diversity of an antibody molecule enlarges astronomically. To induce antigen-antibody response corresponding with the diversity depending on species is reasonable. Thus, a simple induction, production technique and/or manufacturing and a definite demonstration has been expected.

Generally, a substance acting as an antigen is protein and/or bacterial toxin of different species of animals and individuals, protein of virus, proteins such as lipoproteins and glycoproteins of cellular membrane of different species of animals and individuals, lipids such as bacterial cell membrane and polysaccharides and/or lipopolysaccharides of bacterial membrane and blood-type substances. In addition, it has been known that artificially synthesized chemical substance becomes to have a capability of producing antibody against it's substance when the substance is linked to carrier and becomes to be antigenic determinant. A diazonized aromatic amino substrate (dinitrophennyl: DNP, trinitrophenyl: TNP, dinitrochlorobenzen: DNCB et al.) has been used for research as artificial antigen. In other words, when chemical substances and drugs, because of it's low molecule, are linked with a protein (carrier) because of it's low molecule, antibody which is enveloped chemical substance is produced. Thus, the chemical substance itself has not a capability of antibody production, however, when antibody is produced the low molecule substance being able to bind to the antibody when antibody is produced is called hapten. Then, a chemical substance which becomes a new hapten has been expected.

Many improvements are needed to prevent infectious diseases even at the present although peoples have been received the benefit and favor from conventional techniques and clinical application. For example, the influenza which is one of viral infection is related to a primary proliferation on the airway. Moreover, a viral infection such as measles, mumpus and/or chicken pox becomes to onset it's symptom when a virus is proliferated in the blood resulting in expanding into the body after the airway infection. In addition, a virus such as hepatitis and Japanese encephalitis becomes to onset diseases resulting from a proliferation after reaching to the targeted organs via blood stream. Those infections may be able to prevent onset of illness if a neutralizing antibody exists into the blood. Recently, a risk of microbial infection such as HIV, hepatitis virus and/or ebora virus still exists. Prevention of those infectious diseases has been done by immunization injecting a non-pathogenic or inactivating viral antigen against the virus as a preventive method of viral infection. As one of methods, an efficacy of an inactivated vaccine which is made by killed virus and live attenuated vaccine has been well known. Also, an effectiveness of an inactivated vaccine such as Japanese encephalitis virus, hepatitis virus and polyomyelitis virus has been suggested. However, in general, it has been known that an effect of live attenuated vaccine is better than that of an inactivated vaccine. Vaccine against a virus of measles, rubella, mumpus, chicken pox and polyomyelitis has been applied as live attenuated vaccine. Difficult acquisition of antigen such B typed hepatitis and hazardous handling is resolved by antigen, is produced in bacterium resulting from recobination of antigen gene into the bacterial DNA. Recombinant DNA-induced vaccine has been manufactured safely, and has been tested energetically on antibody against HIV. A new type of vaccine such as vector-vaccine and idiotype vaccine is also expected: the former one is to preserve immunity by recombinating gene of a objective virus into a non-pathogenic virus, and later one to utilize anti idiotype antibody which may have an identical structure of the antigen.

Suppurative bacteria (Stapylococcus, Streptococcus peumonia, Pseudomonas aureginosa, Eschericia coli et al.) are treated with immuno-bacteriolysis phenomenon induced by phagocytosis and/or bactericidal action of neutrophils or with activated complement. Bacteria has a substance which resists to phagocytosis on capsule of Diplococcus pneumonia, M-protein of Streptococcus and mucoprotein of Streptococcus, and a dangerous bacteria which has exotoxin such as E. coli 0-157, Shigella, Cholera exists. Toxin produced by those bacilli (tetanus exotoxin and diphtheria exotoxin et al.) is be able to be neutralized by binding with antibody, against those toxins. A major immuno-globulin is antibody belongs to IgG. Also, bacteria such as tuberculosis, leprosy, salmonella, listeria is resistance to bactericidal effect so that neutrophilic cell itself is not able to kill because of it's short-life span. Thus, phagocytosis and sterilization effect of macrophages is needed. Macrophage-activating factor which is one of lymphokaines produced by T cell after responding to antigen is important. And, it's immune bacteriolysis phenomenon affects efficiently on gram-negative bacteria. Namely, in order to the prevention of Neisseria (N. meningococcus and N. gonorrhoeae), this mechanism is an important mechanism. This mechanism is activated by alternative pathway which complement affects on surface substance of bacteria, however, an activation of classical pathway through antibody linked with bacteria (especially IgM. IgG) is more effective. It has been anticipated that new vaccine and antibody against those bacilli is developed. In addition, for example, B-typed hepatitis vaccine is produced by S-antigen of B-typed hepatitis virus, of which materials is used from serum with S-antigen positive subject (HB virus carrier). However, this manipulation is restricted by supply of the materials, has a possible risk of infectious focus from serum and is accompanied with a hazard for the handling. As the means which resolve this problem, by a gene recombination technique, a gene of S-antigen is introduced in microbial DNA such as E. coli and yeast so that a introduced microorganism becomes to produce S-antigen. The method with an availability has been widely developed at the present that when lots of the recombined bacteria are cultured, lots of S-antigen is obtained. This is one of examples in development and application of DNA recombinant vaccine. Also, if amino acid sequence of gene DNA related to antigen is defined, it is technically possible to chemically synthesize antigen-peptides on basis of the sequence resulting in a production of synthesized peptide-antibody. However, immunogenic potency of synthetic peptide alone as a antigen mentioned above is weak so that any kinds of adjuvants is needed to add. Thus, it has been expected a proposal and/or possibility in concrete form and easy procedure, which those structures such as proteins and saccharide chains are able to approximate more closely to an objected antibody.

It is possible to induce antigen of the objective virus on virus which safety has been already confirmed and which is recombinated gene of a targeted idiotype antigen. When the former virus which has confirmed safety is used as vaccine, it is possible to induce an immunity against the later virus at the same time. These vaccines are a kind of vector vaccines so that vaccine which is produced HA-antigen of influenza into vaccinia virus is utilized as a vaccine recombinated antigen-gene of other virus instead of application of idiotype antigen. However, this method is not capable to apply to all kinds of virus although a strain of virus is safe so that it is most difficult to find out vector virus which is preserved a safety adequately against each targeted virus. At an emerging situation, it is hard to supply vaccine temporally, economically and technically. Thus, it has been anticipated to resolve and/or improve those several subjects concerning on conventional vaccine.

Complement plays a role of killing or phagocytosing pathogens resulting from contact directly or indirectly with pathogens. Complement is capable to act directly on bacteria and to be activated by mannose-binding protein. Furthermore, as a result of infection, the produced antibody is able to activate complement so that the activated complement causes an extinction of bacteria or induces immunological phagocytes such as macrophages and/or neutrophils. On bacteria which causes pneumonia or pharyngitis, capsule consisted of long saccharide chain protects a direct action of complement to bacteria. A shape of this mannose-binding protein is altered when it binds to bacteria, resulting in an activation of complement and an promotion of activating phagocytosis. However, although it has known that functional induction and/or generation differs corresponding with different linkage of saccharide chain with a same protein structure, it is very difficult to produce and manufacture glycoproteins with specific action gene-technologically by use of conventional techniques because of biological diversity. It has been anticipated to manufacture and/or demonstrate specific vaccine, antibody, antitoxin and neutralizing antibody which are induced by extracting those species-specific glycoproteins.

And, it is indicated that cells which become tumor have an abnormal metabolism so that there is a possibility of abnormal synthesis of a substance on cell membrane, namely saccharide chain. This abnormality has two possible pathways; a partial synthesis which is not able to synthesize whole length of the saccharide chain, and an extreme different production of saccharides. Those saccharides chain become a tumor-related antigen so that because of short length of molecule, a shortly synthesized saccharide is easily coated the cell surface by a substance and is less effective. Moreover, a possibility has been indicated that substances such as siarylmutin, hyaluronic acid and chondroitin sulfate exist on the surface of tumor cells so that the tumor-related antigen is covered by those substances resulting in dysfunctioning an immune-response. Thus, a proposal of an effective method which increases an induction-rate of tumor-related antigen is expected, accompanied with a trial of prevention and/or treatment against malignant tumors in order to improve an immunological dysfunction due to abnormality of saccharide chain and to scavenge smoothly tumor-induced cells in vivo.

HIV is one of major infectious diseases which has been a world-wide topic. Recently, a treatment depending on only one mechanism of HIV infection has been thought to be difficult to prevent HIV. Generally, virion of HIV (particle of virus itself) is roughly globular and it's diameter is 100 nm. Outer membrane consisted with bilayer of lipid molecule (outer capsule or envelope) is similar to human cellular membrane and identical to evolutional origin. A spike consists of four glycoproteins of gp 120 (gp means glycoproteins). Those proteins of envelope play an important role of binding to cells resulting in invasion into a target cell. A layer of matrix-protein which is called as p 17 exists just below the envelope. Capsid consists of another kind of proteins called as p24. These double strands ofRNA is constituted by about 9,200 base pairs and becomes compact completely into the core of virus. Glycoprotein (gp120) of envelope binds hardly to CD4. Therefore, antibody which recognizes gp120 and gp41 on envelope of HIV binds CD4 as well as those proteins bind a MHC molecule on the surface of healthy cell, resulting in a disturbance of immunological function. Both gp120 and gp41 has a similar amino acid sequence to MHC molecule. It has been developed and tested various kinds of drugs by those biological properties, however, a stage of medicare and/or treatment is not sufficient. Namely, it has been required for a medical prevention to develop a effective vaccine such as an antibody of special glycoproteins of HIV, although it has been speculated that many carriers exist.

Though it is not necessary to do more than to read history of relationship between a man and diseases, a fighting to pathogenic microbacterial infection such as bacteria and virus, it is serious problems which needs a medical resolution. Various kinds of pathogenic microbacteria such as staphylococcus aureus, streptococcus, E. Coli, acid-fast bacteria, mycete and virus usually exist on the living space of human being as origin of various infectious diseases. Thus, lots of drugs are utilized in a treatment and prevention for infectious disease due to pathogenic microorganism and virus. As a result, lots of the benefits have been raised. However, while development of antibacterial agents is noticeable, it has been expected in the field of hygenical and preventative medicine to develop an effective vaccine against wide-spreaded group infections.

It is not sufficient to accept a conventional idiotyped vaccine and/or antibody at the present, although it has been indicated that as a scientific knowledge multidimensional structure of biological substance mentioned above is important to generate various biological functions, and that as an acceptable proposal idiotype vaccine and/or antibody being applied structural diversity on the basis of species-differented cellular specificity in order to be induced functional diversity by multidimensional structure is logical. It is anticipated to be demonstrated a simple method of the simplicity and concrete manufacture and or a performed example. It is a important issue which has been expected as an advanced technology even in the field of a practical medical service which is faced directly on human life as well as in the academic field of biology, chemistry and medicine.

On the other hand, for example, elements of living organism consist of lipids, carbohydrates, proteins, enzymes, nucleic acids, macromolecules such as amino acids and peptides and genes (for example, DNA, tRNA, mRNA, rRNA). By those elements, in addition, formation of cell membrane, intracellular organelle, intracellular and/or extracellular substrates are constructed. Function of these complex substances is generated by multidimensional structure (conformation) depending on each substance. In order to understand the mechanism which is related to development and generation of each physiological function (recognition and/or acceptance of substance), it is important to understand the multidimensional structure which each substance has. (ed. by Alberts, Bray, Lewis, Raff, Roberts and Watson, The Molecular Biology of the CELL, Garland Publishing Inc., 3th. sedition < ref. 2>). A state of charge distribution and electric charge density of molecules which consist of substances and is generated function by these multidimensional structure differs in species differences and morbidity < ref. 2>. In addition, virus, which has not cell membrane and is not living organism, consists of peptides chain which are constructed by many amino acid bindings. And, these virus particles have also multidimensional structure such as two-dimension and/or three-dimension. Among multidimensional structure, helical structure is formed in 3.6 amino acid residues per one helical rotation. Thus, it produces a space which side chain can occupy. And, possible hydrogen bonds on this helical structure can be constituted totally. In addition, multidimensional structure generates the function of a domain, beta domain, a /beta domain, exon or intron. This concept is also a scientific fact and important knowledge. Though a core part of this structure is conserved in homologous proteins, dimensional changes in a helix loop region occur. Moreover, formation of conformation depends on a type of the secondary structure to bind each loop and a number of amino acids in helix loop rather than amino acid sequences. Therefore, it is in general to be determined by combination of α - α, beta-beta, α - beta or beta sheet- a loop. These multidimensional structure is to apply to molecular biology of every kinds of genes and antibodies from recent knowledge. It is well known scientific fact and knowledge that it is important to generate physiological function based on recognizing two- or three-dimensional conformation of each substance. It has been raised to produce and/or synthesize an artificial substance which responds specifically against each substance, as well as to exactly resolve a function of organism. And, it has been anticipated to develop a method related on treatment and/or medical care on the basis of a knowledge concerned on biological functions of organism with a diversity and species-specificity, resulting from integrating many combinations with those substances.

On a biological fact mentioned above, it has been expected to develop vaccine precursor or vaccine, for the manufacture of an antitumor agent, as a highly selective or specific product.

An object of this invention is to resolve the problems mentioned above and to provide vaccine precursor or vaccine or the manufacture of an antitumor agent.

And, those providing substances and/or compounds are produced and/or manufactured by a simple technique, is similar to the origin of generating a diversity of species such as a specificity and/or selectiveness as far as possible, and has a property of more discriminating a targeted acceptor-substance.

On the basis of effects of granulizing and dispersing cells as antitumor agent which antigenic inducers described below have, it is possible to produce and/or manufacture the above mentioned vaccine precursor or vaccine.

### CONSTITUTION OF THE INVENTION

The invention claimed in claims 1-4 in order to attain the above-mentioned objective is the use of a vaccine or a vaccine precursor obtained by in vitro treating a pathogen or cells of pathological tissues with an antigenic substance inducer for the manufacture of an antitumor agent.

A fundamental principle of this invention is to aim to obtain a capacity of antibody recognition to organism by utilizing granulized and/or fragmented structural substance as a result of extincting tumor (cancer) cells resulting from an application of quantum thermodynamic disruptive and micronizing effects of antigenic inducers. Antigenic inducers mentioned in this invention have been disclosed already in International Publication WO96/07403 related to the aspect on inhibitory or blocking agents of molecular generating and/or inducing functions as well as synthesis process thereof.

The antigenic substance inducer used in the present invention contains as an affective ingredient the compound represented by the Formulae below.

Formula 3-a recited in claim 1 is 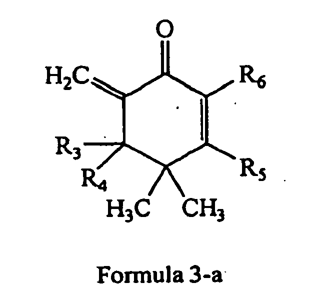 (wherein
(i) R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group; (ii) one or more of R3 and R4, and/or one or more of R5 and R6 may be substituted or non-substituted cyclopentyl group; substituted or non-substituted cyclohexyl group; or substituted or non-substituted naphthyl group; (iii) R5 and R6 may form a ring by binding with another condensation polycyclic hydrocarbon compound or heterocyclic compound; (iv) one or more of R3, R4, R5 and R6 may be substituted by one or more of substituents selected from the group consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamino group, C1-C6 acyloxy group, C2-C6 alkenyl group, C1-C6 trihalogenoalkyl group, C1-C6 alkylamino group, and C1-C6 dialkylamino group; (v) R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group; (vi) when one or more of R3, R4, R5 and R6 are alkyl groups, terminal end(s) of the alkyl group(s) may be substituted by C3-C8 cycloalkyl group).

In Formula 3-a, aryl group in (i) and (iv) mentioned above may be phenyl, tolyl, xylyl or naphthyl group. The substituted cyclopentyl group in (ii) mentioned above may be cyclopentylamino group or cyclopentylcarbinol group. The substituted cyclohexyl group mentioned above may be cyclohexylamino group, cyclohexylaldehyde group or cyclohexyl acetic acid group. The substituted naphthyl group mentioned above may be naphthyl amino group or a naphthylamino sulfonic acid group. Condensation polycyclic hydrocarbon compounds in (iii) mentioned above may be pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyclooctene. The heterocyclic compound mentioned above may be furan, thiophene, pyrrole, γ - pyran, γ-thiopyran, pyridine, thiazole, imidazole pyrimidine, indole or quinoline.

Formula 3-b recited in claim 2 is 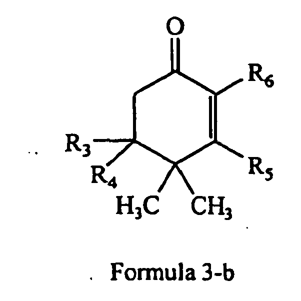 (wherein
(i) R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group; (ii) one or more of R3 and R4, and/or one or more of R5 and R6 may be substituted or non-substituted cyclopentyl group; substituted or non-substituted cyclohexyl group; or substituted or non-substituted naphthyl group; (iii) R5 and R6 may form a ring by binding with another condensation polycyclic hydrocarbon compound or heterocyclic compound; (iv) one or more of R3, R4, R5 and R6 may be substituted by one or more of substituents selected from the groups consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamino group, C1-C6 acyloxy group, C2-C6 alkenyl group, C1-C6 trihalogenoalkyl group, C1-C6 alkylamino group, and C1-C6 dialkylamino group; (v) R5 may be substituted by one or more substituents selected from the group consisting of halogen atom, C1-C6 alkyl group, protected or non-protected carboxyl group; protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group; (vi) when one or more of R3, R4, R5 and R6 are alkyl groups, terminal end(s) of the alkyl group(s) may be substituted by C3-C8 cycloalkyl group).

In Formula 3-b, aryl group in (i) and (iv) mentioned above may be phenyl, tolyl, xylyl or naphthyl group. The substituted cyclopentyl group in (ii) mentioned above may be cyclopentylamino group or cyclopentylcarbinol group. The substituted cyclohexyl group mentioned above may be cyclohexylamino group, cyclohexylaldehyde group or cyclohexyl acetic acid group. The substituted naphthyl group mentioned above may be naphthyl amino group or a naphthylamino sulfonic acid group. Condensation polycyclic hydrocarbon compounds in (iii) mentioned above may be pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyctooctene. The compounds of heterocyclic group mentioned above may be furan, thiophene, pyrrole, γ-pyran, γ -thiopyran, pyridine, thiazole, imidazole pyrimidine, indole or quinoline.

The mechanism of action and/or logical explanation of each antigenic substance inducer mentioned above has been already disclosed in International Publication No. WO96/07403. This invention indicates that a manipulation with each antigenic substance inducer is able to induce an action of more specific recognition which is mentioned below.

It is easy to consider a possibility to produce monovalent vaccine, combined vaccine (which consists of various types of cells), cross-reactive vaccine according to an expected effect from various pathological tissues and/or organs which are disturbed by tumor cells, or from pathological tissues. Thus, antigenic substance inducer mentioned in this invention does not disturb a fundamental structure depending on species resulting in a production and/or induction of multiple antibodies according to biochemical components which are closed evolutionary to species, and resulting in a production and/or induction of multifunctional antibodies (a diversity of antibody recognition) which are able to recognize multiple antibodies. It is of course that when antigenic substance inducer (preventative dosage or less than lethal dosage) is given at an onset point of tumor (cancer) in vivo, these constituted elements (granules and/or particles) extincted cells become to stimulate immune system of the host and to produce an antibody, so that further proliferation and invasion in vivo as a circulus vitiosus is blocked by this positive feed-back mechanism. It is to amplify an inactivated vaccine in vivo. Those techniques are able to be applied into preventive techniques of a disease. It is of course to be able to apply on substances of non-organism as a kind of antibody catalysts.

Moreover, each extracted substance followed by a processes mentioned in this invention is able to be added a further detoxification by ultraviolet irradiation, heating and/or conventionally used organic solvents such as aldehyde.

One or both terminals of each structure (mainly, amino terminal and carboxyl terminal) of vaccine precursor or vaccine which is manufactured and/or produced by antigenic inducer mentioned above is substituted by more than one substituent which is chosen from substituent group consisting of halogen atom, cyano group, protecting carboxyl group, protecting hydroxyl group, protecting amino group, protecting alkyl group, protecting alkoxy group, protecting alkoxy carbonyl group, protecting aryl group, protecting cycloalkyl group, protecting acylamino group, protecting acyloxy group, protecting C2-C6 alkenyl groups, protecting C1-C6 trihalogenoalkyl group, protecting C1-C6 alkylamino group, protecting C1-C6 dialkylamino group, protecting C1-C6 aminoalkyl group, protecting C1-C6 alkylamino C1-C6 alkyl group or protecting cycloamino C1-C6 alkyl group.

In the present specification, unless otherwise specified, the term "halogen atom" means, for example, fluorine atom, chlorine atom, bromine atom or iodine atom; the term "alkyl group" means C1-10 alkyl group such as methyl group, ethyl group, n-propyl group, isopropyl group, n-butyl group, isobutyl group, sec-butyl group, tort-butyl group, benzyl group, hexyl group, octyl group or the like; the term "lower alkyl group" means C1-5 alkyl group among the alkyl groups mentioned above; the term "alkoxy group" means -O-alkyl group (alkyl group is C1-10 alkyl group mentioned above); the term "lower alkylamino group" means C1-5 alkylamino group such as methylamino group, ethylamino group, propylamino group or the like; the term "di-lower alkylamino group" means C1-5 dialkylamino group such as dimethylamino group; the term "lower alkenyl group" means C2-5 alkenyl group such as vinyl group, allyl group, 1-propenyl group, 1-butenyl group or the like; the term "cycloalkyl group" means C2-6 cycloalkyl group such as cyclopropyl, cyclobutyl, cyclopentyl, cyclohexyl or the like; the term "aryl group" means, for example, phenyl group or naphthyl group; the term "alkoxy carbonyl group" means -COO-alkyl group (alkyl group means C1-10 alkyl group above mentioned); the term "hydroxy lower alkyl group" means hydroxy-C1-5 alkyl group such as hydroxy methyl group, hydroxy ethyl group, hydroxy propyl group or the like; the term "amino lower alkyl group" means amino C1-5 alkyl group such as aminomethyl group, aminoethyl group, aminopropyl group or the like; the term "lower alkylamino lower alkyl group" means C1-5 alkylamino C1-5 alkyl group such as methylaminomethyl group, ethylaminomethyl group, ethylaminoethyl group or the like; the term "di-lower alkylamino lower alkyl group" means C1-5 dialkylamino C1-5 alkyl group such as dimethylaminomethyl group or diethylaminomethyl group; the term "cyclic amino group" means cyclic amino group with 4-10 membered ring such as piperazinyl group, morpholinyl group, 1,4-diazabicyclo (3,2,1) octyl group or the like; the term "cyclic amino lower alkyl group" means C1-5 alkyl group attached to cyclic amino group with 4-6 membered ring such as 1-piperazinylmethyl group, 1-pyrrolidinylmethyl group, 1-azethydinylmethyl group, 1-morpholinylmethyl group or the like; the term "acylamino group" means C1-4 acylamino group such as formylamino group, acetylamino group, propionylamino group, butyrylamino group or the like; the term "acyloxy group" means C1-4 acyloxy group such as formyloxy group, acetyloxy group, propionyloxy group, butyryloxy group or the like; the term "trihalogeno-lower alkyl group" means trihalogeno C1-5 alkyl group such as trichloromethyl group, trifluoromethyl group or the like; the term "heterocyclic group" means 5 membered ring, 6 membered ring or those condensation rings (such as furyl, propyl, thienyl, oxazolyl, imidazolyl, thiazolyl, 1-pyrrolinyl, benzofuryl, benzothiazolyl, pyridyl, quinolyl, pyrimidinyl or morpholinyl group as an example) which has one or more atoms selected from the group consisting of oxygen atom, nitrogen atom and sulfate atom.

Protecting group of carboxyl group may be a carboxyl-protecting group which is pharmaceutically acceptable, such as ester-forming group which is seceded easily from an organism.

Moreover, protecting group of amino group, amino lower alkyl group and lower alkylamino lower alkyl group may be a protecting amino group which is pharmaceutically acceptable and which is seceded easily from an organism.

In addition, protecting group of hydroxyl group and hydroxy lower alkyl group may be a protecting hydroxyl group which is pharmaceutically acceptable and which is seceded easily from an organism.

In order to transport to each targeted organ and/or tissue and to effectively generate each targeting effect, for example, carrier substances which are pharmaceutically acceptable may be combined.

The carrier substances which are pharmaceutically acceptable may include the carrier substances which are biologically acceptable, such as polyoxyalkylenealkyl ether, polyoxyethylene sorbitan fatty acid ester, polyvinylpyrrolidone, hydrocarbon, paraffin, alcohol, polyvalent alcohol, alcohol ester, polyalcohol ester, fatty acid and metal salts of fatty acid, artificially produced liposome, with mixture, homogeneous diffusion and/or dispersion.

In addition, when vaccine precursor or vaccine are used as combined substances with the carrier substances which are allowable on pharmaceutic use, these can be applied in many kinds of the generally known agent types such as milky lotions, suspensions or solutions corresponding to prospected treatments. Also, it is allowed to add solvent supporting agents, isotonic changing agents, pH adjusters, deodorants, antiseptics or odorants in the compounds mentioned in this invention.

As a method of extracting antigenic substance from a cancer cell, for example, it is possible to be extracted by washing with physiological saline and/or with Triton detergent.. And, a combination of manipulation with an ultrasonic wave and frozen processing is also available as a conventional method of cellular destruction. Antibody titer can be increased by adding an adjuvant such as A1(OH)₃ after treating antigen-included solution which was obtained by a centrifuged separation with heating, formaldehyde and/or ultraviolet irradiation for an inactivation.

An inactivation of vaccine is possible to perform utilizing a typical and fundamental processing which has been applied (ref. [Provost, P. J., Proc. Soc. Exp. Biol. Med. 160,213 (1979); Provost, P. J., J. Med. Virol. 19,23 (1986)]). Each objective extracted substance is more inactivated by heating, pH modification, irradiation and, by treatment with an organic solvent such as formalin or paraformaldehyde. However, a non-treated extract may be possible to apply.

As a method of extracting antigenic substance from a cancer cell, for example, it is possible to be extracted by washing with physiological saline and/or with Triton detergent. And, a combination of manipulation with an ultrasonic wave and frozen processing is also available as a conventional method of cellular destruction.

Antigen-included solution which was obtained by a centrifuged separation is used as original antigen or is treated with heating, formaldehyde and/or ultraviolet irradiation for,an inactivation so that it is possible to produce vaccine which is added a stabilizing agent consisted of the following Table 1.

**Table 1**

| | |
|---|---|
| Lactose | 2.5% (w/v) |
| Glucose | 2.5% (w/v) |
| Human Albumin | 0.2% (w/v) |
| Gelatin | 0.3% (w/v) |
| Sodium Chloride | 8.0 g |
| Sodium Phosphate (dibasic) | 1.15 g |
| Sodium Phosphate (monobasic) | 0.2 g |
| Potassium Phosphate | 0.2 g |
| Distilled Water | 1 liter |
| pH | 7.2 |

Also, it is possible to be additionally or supplementarily inactivated each objective extracting substance by heating, pH modification, irradiation or a processing with an organic solvent such as formalin or paraformaldehyde after washing sediments of culture medium which is extincted each cell or bacteria. When it is treated with formaldehyde, a desirable suspension concentration is 0.001- 0.05 (v/v) %. When it is inactivated by ultraviolet irradiation, a desirable range is 5.0 × 10 J/m²-5.4 × 10⁴ J/m² at 356 nm. However, it is better to be not used unless it is needed. A desirable temperature when formaldehyde or ultraviolet irradiation is applied is maintained below 4°C. Anyway, an increased degree of heating, formaldehyde or ultraviolet irradiation was to change a titter and/or specificity markedly. For example, a titer of immunoglobulin obtained by agar-gel became less than 1/100. However, an original substance alone in this invention provided a more efficiency to generate an absorption of antigen particle and a clustering. In addition, an antibody titer is increased by adding a surfactants (Tween 80).

Moreover, a property as an adjuvant or carrier is able to be increased by an absorption of an inactivated extract to aluminum hydroxide or by a precipitation in aluminum hydroxide. For example, firstly, sulfuric-acid potassium aluminum is added into a bulk solution which is treated with Yoshixol or which is inactivated by formalin resulting in a production of direct sediment of inactivated extract in aluminum hydroxide, so that a final sediment is produced by addition of sodium hydroxide. It is possible to replace on physiological saline, removing formaldehyde and residual salt from a suspension.

In addition, an objective substance is extracted organic-chemically after washing sediment of culture medium with extincted cells mentioned above as similar as an extracted solution of conventional vaccine. For example, an extraction is performed by an addition of mixture with a bubble- or form-removing agent such as chloroform and isoamylalchol, and an extracted solution is flowed DEAE Sephadex gel with 0.1 N hydrochloric acid into a column which has prepared with 20 mM phosphate buffer solution (pH 7.5), sodium chloride solution of 2g/liter with 2% formalin and 1M sodium chloride solution. In order to make an equilibrium of a culumn and to perform a chromatography, pH and concentration of osmotic pressure is completely balanced by flowing a phosphate buffer solution of pH 7.5 (20 mM phosphoric acid and 0.1% Tween 80) so that a flowed sample is adjusted an osmotic pressure concentration within a range of 280 mOsm. Then, an infusion rate of a phosphate buffer solution of pH 7.5 (20 mM phosphoric and 0.1% Tween 80) is set on 45 cm/h. An antigenic substance is isolated as a soluble one by this manipulation. It is possible to condense antibody fraction which is obtained by gel-filtration column under identical condition to condensed manipulation mentioned above. A final liquid volume after the rinse is adjusted to 2.5 liter. A refined and collected sample is obtained by filtering a condensed antibody fraction through a membrane (Durapore) with a pore of 0.2 micrometer.

Moreover, as a method of another gel-filtration, a final processing of gel-filtration chromatography is performed after a negative ion exchange chromatography. For example, it is a column (K215/100; Pharmacia LTD) which is composited 26.5 liter of Sepharose 6BCL. A whole system of column apparatus, pipe-line and detector is sterilized by solution of 2% formalin and 2% sodium chloride with a flow rate of 1.7 litter/h so that a culumn is rinsed and balanced by pH 7.5 phosphate buffer solution (20 mM phosphoric acid and 0.1% Tween 80) with a flow rate of 2.7 liter/h for 48 hours. A condensed antigenic substance is added gravitionally into a column so that it is filtered at a flow rate of 2.7 liter/h with pH 7.5 phosphate buffer solution (20 mM phosphoric acid and 0.1% Tween 80), obtaining a refined and collected sample.

As a solvent for extraction, it is possible to use a halogenated C1-C6 alkane such as metylenechloride and tetrachloroethane as well as chloroform. In addition, a negative ion exchanger matrix which is used in a extraction processing due to an ion exchanger chromatography is not qualified and is demonstrated a following material: DEAE cellulose, DEAE agarose, DEAE biogel, DEAE dextrane, DEAE Sephadex, DEAE Sepharose, aminohexyl Sepharose, ECTEOLA cellulose, TEAE cellulose, QAE cellulose, mono-Q or benzyldiethylaminorthyl cellulose.

Vaccine precursor or vaccine which are used by this invention, can be completed above-mentioned objectives by use of the compound alone, and can be utilized together with acid addition salts, emulsifiers, ester agents or polymerization agents, unless effectiveness is changed drastically. It can be used in the following form as an example of acid addition salts: non-toxic salts which are allowable pharmaceutically, and are inorganic acids such as hydrochloric acid, hydrobromic acid, phosphoric acid or sulfuric acid and organic acids such as acetic acid, citric acid, tartaric acid, lactic acid; succinic acid, fumaric acid, maleic acid or methasulfonic acid.

When vaccine precursor or vaccine which is produced by antigenic inducer is used antitumor (anticancer) agent, it is possible to be administered as a single agent or a combined agent with the carrier substances which can be allowable as drugs. When a pain may be induced by an administration such as subcutaneous injection, it is possible to add a local anesthetic such as xylocaine to the objective solution.

These compositions are dependent on routes and/or planning of administration.

When vaccine precursor or vaccine, which is produced by antigenic inducer which is shown in this invention, are used as antitumor (anticancer) agent those can be administered orally or non-orally as medicament compositions such as powders, granules, tablets, capsules, injection solutions by suitably mixing with adequate components such as carrier substances, excipients or attenuants which are allowable pharmaceutically.

The vaccine precursor or vaccine which is produced by antigenic inducer are used for the manufacture of an antitumor (anticancer) agent, by oral route, several types of tablets, capsules, powder materials, granular agents and liquid agents are available. When the compound is administered through the non-oral route, those are used in the form of disinfected fluid. When the compounds are used as types above-mentioned, the carrier substances with nontoxic solids or fluids include in a composition.

As an example of solid carriers, capsules made by usual gelatin is used. Moreover, effective ingredients are utilized with subsidiary substances or by tabulating, granulating and/or powder packaging without subsidiary substances. The following substances are used as the excipients; gelatin, lactose, sugars such as glucose, corn, wheat, rice, starches such as corn starch, fatty acids such as stearic acid, fat bases such as calcium stearic acid and magnesium stearic acid, talc, vegetable oil, alcohol such as stearylalcohol and benzyl alcohol, gum, polyethylene alkylene glycol and so on.

These capsule, tablet, granule and powder are generally 0.1-80 weight % and contains effective ingredient of 0.1-60 weight %. Liquid carriers such as water, physiological saline, sugar solution, dextrose solution, ethylene glycol, propylene glycol, glycols such as polyethylene glycol, polyoxyethylene sorbitan monoolate are desirable.

When it is administered non-orally by the manner of intramuscular injection, intravenous injection or hypodermic injection, the compounds provided in this invention are used as the germ-free solution which is added other solutes such as minerals or glucose in order to make the isotonic solution. Appropriate solvents for an injection represent sterilizing water, solution of lidocaine hydrochloride (for intramuscular injection), physiological saline, glucose solution, any kind of fluids for an intravenous injection, electrolyte solution (for intravenous injection) and so on. When those solutions for the injection are used, usual dosage is 0.01-20 weight % and is desirable at 0.05-5 weight %.

In the case of liquids for oral administration, it is better to be used as suspension or syrup with 0.01-20 weight %. A carrier of these liquids is watery excipient such as perfume, syrup and micelle which are available for pharmaceutic manufacturing.

The chemical compound which is used and enforced in this invention is not especially limited. But, as one of a concrete and representative compound which shows reasonable biological effect and which is easily synthesized chemically because of the simple chemical structure, it was synthesized 4,4-dimethyl-6-methylene-2-cyclohexen-1-one (this compound is termed as Yoshixol) which is the compound wherein all of the substituents R3, R4, R5 and R6 in Formula 3-a are hydrogen atoms. And, unless otherwise specified, representative experiments are demonstrated for showing effectiveness of this invention by using this Yoshixol.

### BRIEF DESCRIPTION OF THE DRAWINGS

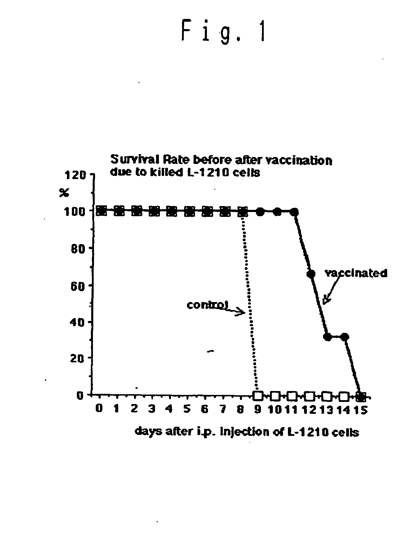

Fig. 1 is a graph which is evaluated a survival rate of mouse leukemia L1210 as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

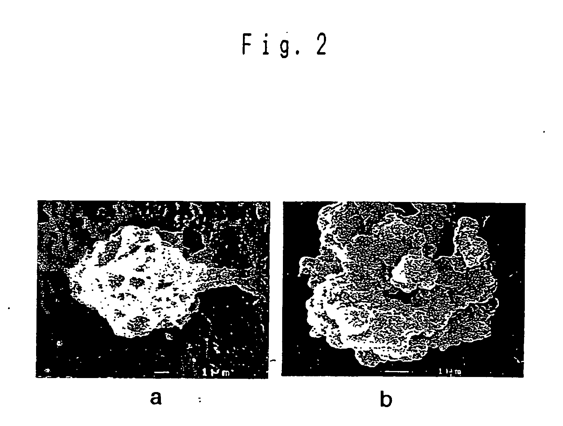

Fig. 2a is an electron microscopic aspect of mouse leukemia L1210 before treatment with antigenic substance inducer which is mentioned in this invention. Figure 2b is a extincted aspect of mouse leukemia L1210 after treatment with antigenic substance inducer. A fine small particle with membrane components such as proteoglycans is demonstrated.

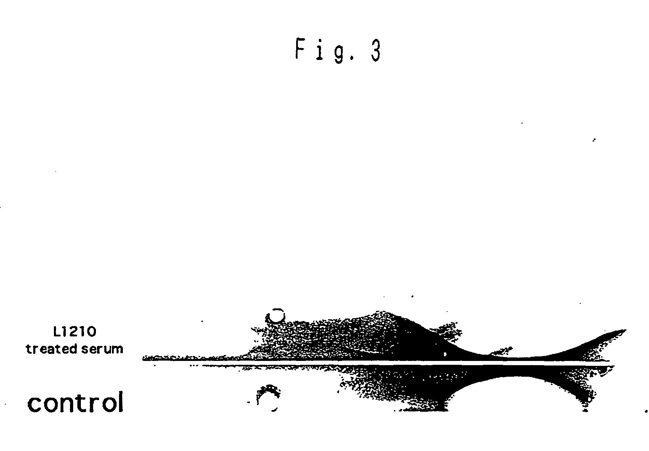

Fig. 3 is a graph of immuno-electrophoresis which demonstrate to induce a new production of antibody in rabbit serum against mouse leukemia L1210 as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

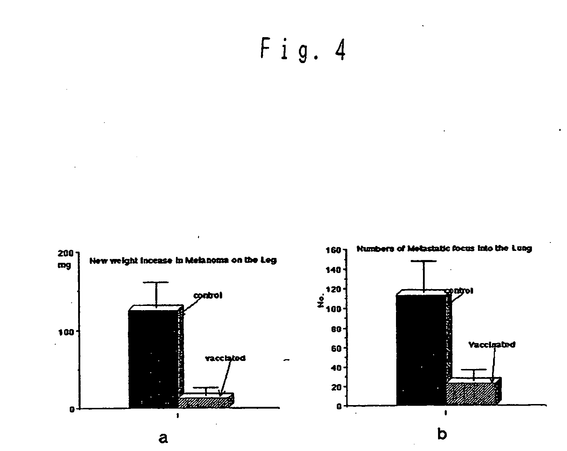

Fig. 4 is the graph which is evaluated changes in tumor weight of melanoma cells B16 implanted to foot-pat and a number of colony formation in lung as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

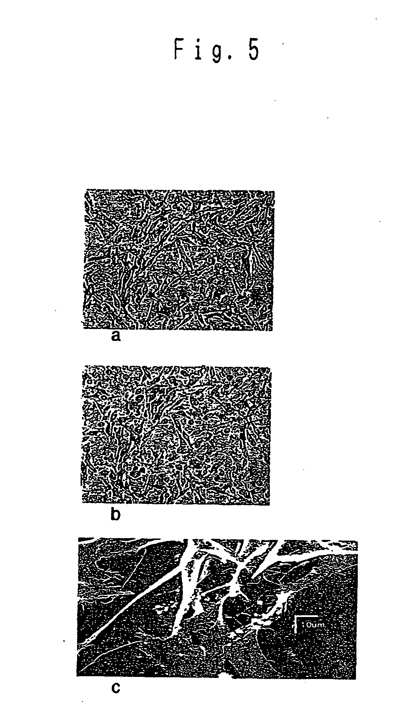

Fig. 5 show an investigated morphological aspect of mouse melanoma cell B 16 before and after treatment with antigen structure inducer mentioned in this invention. Figure 5a is an aspect of phase-contrasted microscopy without the treatment. Figure 5b is an aspect of phase-contrasted microscopy with the treatment. Figure 5c is a scanning electron microscopic aspect.

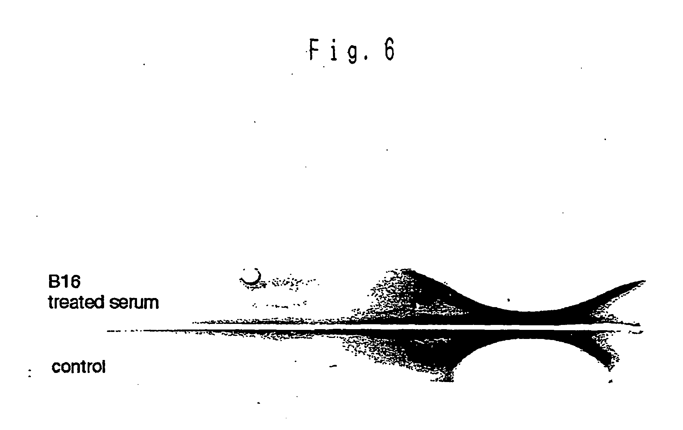

Fig. 6 is a graph of immuno-electrophoresis which demonstrate to induce a new production of antibody in rabbit serum against mouse melanoma cell B16 as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

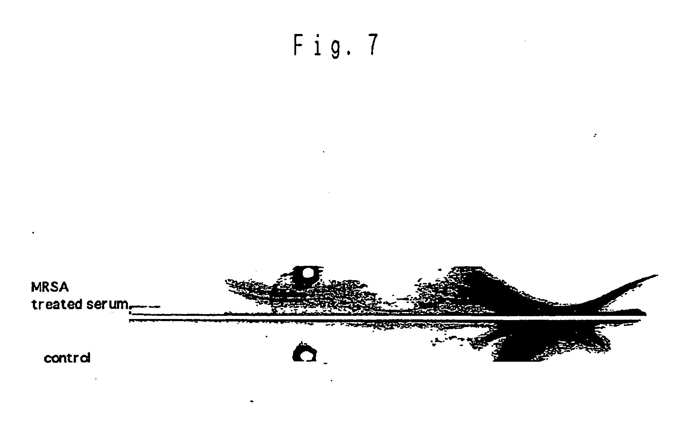

Fig. 7 is a graph of immuno-electrophoresis which demonstrate to induce a new production of antibody in rabbit serum against methicilin resistant Staphylococcus aureus as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

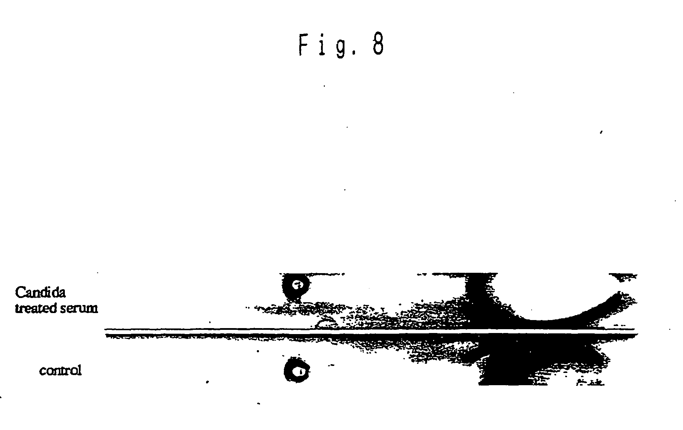

Fig. 8 is a graph of immuno-electrophoresis which demonstrate to induce a new production of antibody in rabbit serum against Candida albicans as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

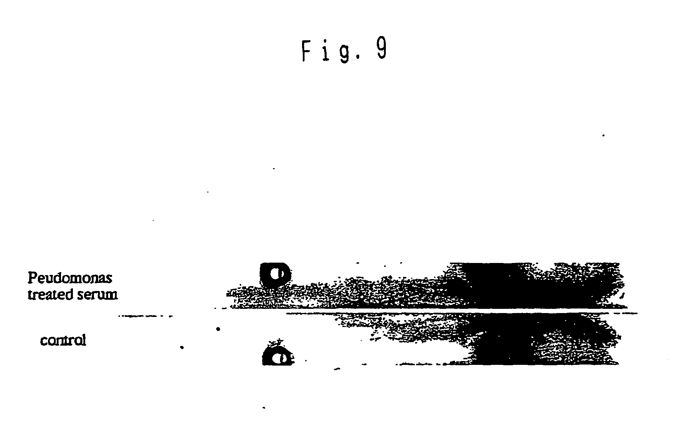

Fig. 9 is a graph of immuno-electrophoresis which demonstrate to induce a new production of antibody in rabbit serum against Pseudomonas aureginosa as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

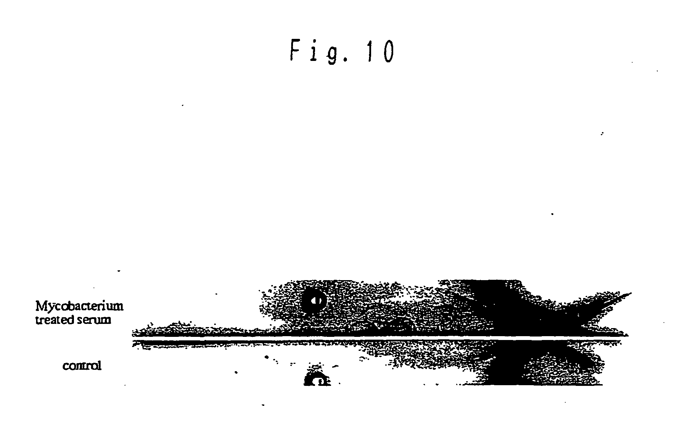

Fig. 10 is a graph of immuno-electrophoresis which demonstrate to induce a new production of antibody in rabbit serum against Mycobacterium rapid grower as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

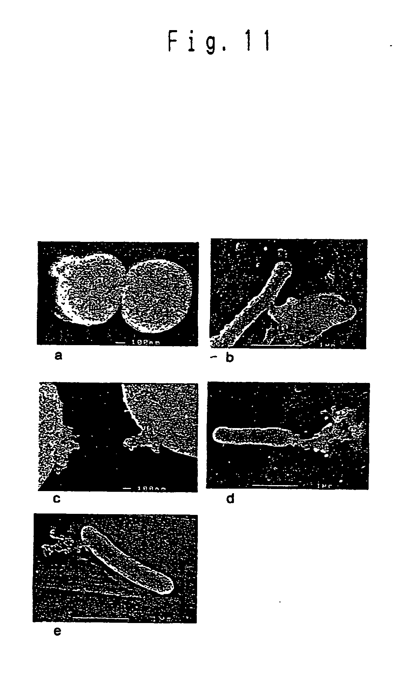

Fig. 11a shows an electron microscopic aspect of methicilin resistant Staphylococcus aureus treated with antigenic substance inducer mentioned in this invention. Each of Figure 11b, 11c, 11d, and 11e shows a extincted aspect investigated by an electron microscopy on E. coli, Candida albicans, Pseudomonas aureginosa and Mycobacterium rapid grower, respectively. Each picture demonstrates an appearance of a small particle with membrane components of each bacteria.

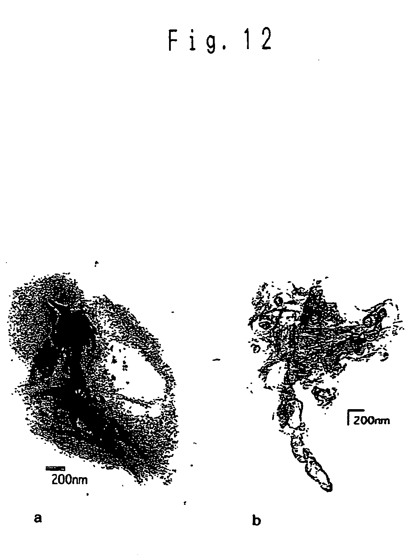

Fig. 12a is a transmission electron microscopic aspect of extincted methicilin resistant Staphylococcus aureus after treatment with antigenic substance inducer mentioned in this invention. Figure 12b is a transmission electron microscopic aspect of extincted E. coli. A fine small particle with membrane components such as peptidoglycans or proteoglycans is demonstrated.

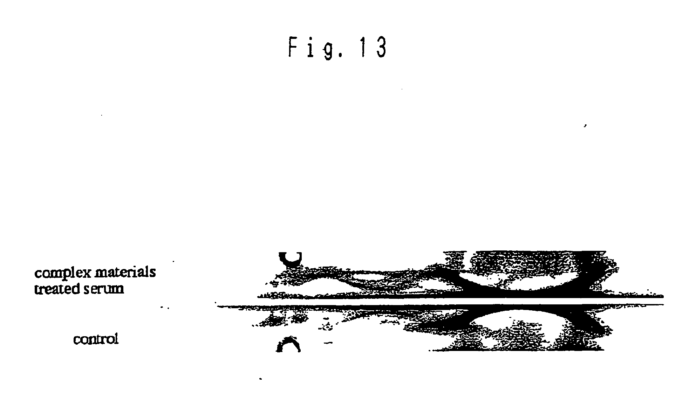

Fig. 13 is a graph of immuno-electrophoresis which demonstrate to induce a new production of antibody in rabbit serum against a mixed solution with chondroitin sulfate, fibrinogen, actin and insulin as an example of effect of vaccine precursor, vaccine, antibody, neutralizing antibody, antitoxin and idiotype antibody which is produced by use of antigenic substance inducer in this invention, and anti-idiotype antibody which is produced by the idiotype antibody.

### EMBODIMENTS OF THE INVENTION

Mouse leukemic cells L-1210 (Cancer research institute, Tokyo) was incubated with 2 ml of culture medium (Minimum Essential Medium, Gibco Co.) including bovine fetus serum (Dai-Nipon Pharmace. Co.) at a temperature of 37 °C and 5% CO₂ for 30 hours in the incubator. When a number of cell becomes to around 1x10⁶, cell death is induced by an addition of 4 µl of Yoshixol which concentration is 2M with ethanol. As a result, a determination of cell viability due to an uptake of methylene blue showed a survival cell of 5x10⁴ 2 hours after Yoshixol, closely zero level after 20 hours and zero after 30 hours. After confirming extinction of cells in culture medium, the culture medium which contains extincted cells was centrifuged at 3,000 cycles/min for 5 minutes so that it's supemant was removed. After adding physiological saline of 0.9 cc into the sedimented component and stirring, a centrifuging separation was performed at 1,000 cycle for 5 minutes. This procedure was repeated twice. Then, after adding physiological saline of 0.9 cc in the rinsed reside of sediment and stirring, the mixtured solution was filtered by miripore-filter of cellulose acetate with 0.45 micrometer pore. This filtered solution was injected intraperitoneally at a range of 0.2 cc/mice into CDF-1 strain of male mice (Charles-River Co, Saitama). On 31 days after the injection of the filtered solution, 0.3 cc of culture medium (1x10⁶ cells included) which is already cultured was injected intraperitoneally in control group which has not injected the solution and in treated group which has injected the solution so that a difference of timing of death due to progression of leukemia was investigated. Subsequently, as shown in Figure 1, all mice in control group was dead within 8 days after transplantation of cells. In contrast, mice which was treated the filtered solution became to start death on 12 days after the transplantation. Longest survived period was prolonged to 14 days after the transplantation. This result indicates that by a production of antibody as antigen due to residual substance which cell has been destroyed by Yoshixol, it is possible to induce to prolong a survival period of mice in leukemia.

Morphological changes appeared after treatment with Yoshixol on mice leukemic cell were investigated by scanning electron microscopy (Nihon Densi Co. JSM-6000F). In non-treated group, cultured cells have a irregular pattern such as doughnut-like shape with a central pore which seems to be a nose of pig, sponge-like structure and spikes. It seems to be an aspect of " nonfeeling " (Figure 2-a). In the contrast, in treated group which is added 10 ul of Yoshixol into the cultured medium, there were a various types of a destroyed many cells. An appearance of adhesion and aggregation between cells did not find so that a morphological aspect is identical to apoptosis or natural cell death (Figure 2-b). Thus, these findings suggest that a particle of fragmented cellular component (10-100 nm) such as proteoglycan acts as antigen resulting in easy phagocytosis of macrophages and lymphocytes so that those particles is involved in a physiological immune surveillance mechanism in vivo.

On an evaluation of survival period of mice mentioned above, a pathological examination was performed at a timing of the death light-microscopically. An observation was done after being stained by HE for each section of heart, liver, kidney, lung, spleen and intestine which has been embed by paraffin and fixed by 10 % formaldehyde solution. In non-treated group, an invasion of leukemic cell was found markedly on each organ. Additional histological findings were portal dilation and stagnation and destroy of vascular wall in liver, and destroy of alveoli and nodule formation from vessels in lung. Also, bloody ascites was found characteristically. On the other hand, in the treated group which has been sensitized, a histological aspect was similar to that in non-treated group, however, a degree of each characteristic finding was less than that in non-treated group. Except for a bloody ascites, a qualitative finding is similar between both groups although a histological aspect has been observed at postmortern. This result suggests that at least treatment with vaccination mentioned in this invention is related to biological mechanism of inhibiting to induce a blood ascites (possibly, damage of vascular wall) due to transplanted leukemic cells.

Mouse leukemic cells L-1210 purchased from Cancer research institute, Tokyo, was incubated with 2 ml of culture medium (Minimum Essential Medium, Gibco Co.) including bovine fetus serum (Dai-Nipon Pharmace. Co.) at a temperature of 37°C and 5% CO₂ for 30 hours in the incubator. When a number of cell becomes to around 1x10⁶, cell death is induced by an addition of 4 µl of Yoshixol which concentration is 2M with ethanol. As a result, a determination of cell viability due to a uptake of methylene blue showed a survival cell of 5x10⁴ 2 hours after Yoshixol, closely zero level after 20 hours and zero after 30 hours. After confirming extinction of cells in culture medium, the culture medium which contains extincted cells was centrifuged at 3,000 cycles/min for 5 minutes so that it's supemant was removed. After adding physiological saline of 0.9 cc into the sedimented component and stirring, a centrifuging separation was performed at 1,000 cycle for 5 minutes. This procedure was repeated twice. Then, after adding physiological saline of 0.9 cc in the rinsed reside of sediment and stirring, the mixtured solution was filtered by miripore-filter of cellulose acetate with 0.45 micrometer pore. This filtered solution of 1 cc was injected intravenously in a rabbit. One month after the injection, the rabbit was sacrificed by bleeding under Nembutal anesthesia so that the serum was obtained after removal of cell components by a centrifugation. This serum was examined by an agar-gel diffusion of rabbit serum antibody against goat serum (Jackson ImmunoResearch Lab, PA, USA) in order to define whether this serum has been produced an antibody (immunoglobulin) against mice leukemic cells. Subsequently, it was confirmed to be formed a new band of immunoglobulin as shown in Figure 3.

In addition, rabbit serum as the control which was shown in this example was produced by the following. In a japanese white rabbit which was born from a parent as same as a rabbit which was used in the above example, 10 ul/Kg of 2 M Yoshixol with ethanol was injected intravenously for 3 min via a ear vein. Solution of Yoshixol was mixed with each culture solution or culture medium of 5 ml and was filtered through 0.45 um miliporefilter of cellulose acetate. Any abnormal sign did not find immediately after the injection through one month. A rabbit was sacrificed under Nembutal anesthesia one month after the injection so that the serum was obtained after removal of cell components by a centrifugation.

Moreover, the immune electrophoresis which was demonstrated in this invention was performed the following procedure. However, this invention is not restricted by the investigative procedure. Firstly, 1% agar-sol was made by heating and melting 1g of agar-agar (Wako Pure Chemical LTD.) with 100 ml of 0.03 M Veronal buffer solution. Veronal buffering solution (pH 8.6) was prepared by a mixture of 5,5-diethylbarbituric acid (Wako Pure Chemical LTD.) and 5,5-diethylbarbituric acid sodium (Wako Pure Chemical LTD.), Sodium nitride (Wako Pure Chemical LTD.). As a procedure, firstly, agar-plate was made on a glass plate (10 cm x 10 cm) cleared with ethanol gauze after pouring agar-sol. Original core point and a groove which are added each serum is planing on a paper graph so that the paper was set on a agar-gel plate after being harden the agar-sol. Then, a part of agar-gel was removed in order to obtain a hole for sample. Thereafter, a 4 ul sample of rabbit serum which is added bromphenol blue (Wako Pure Chemical LTD.) was replaced into the sample hole. And, electrophoresis was performed by an electrophoretic apparatus (power supply ATT0-Power Station 1000VC) with a constant current of 2-3 mA for about 18 hours after replacing the gel in the chamber following a method of Ouchterlony. Then, after taking the gel off from the chamber, a part of gel which has been marked previously as a groove was removed. In this groove, goat serum against rabbit of 58 mg/ml was added so that it was reacted at a room temperature for an overnight. It was preserved in the humidifier box in order to prevent drying. After the preparation, the gel was dipped in a physiological saline for one day. In order to remove dust and contamination, the gel was dipped in an evaporated water. The gel was stained by CBB staining solution (Wako Pure Chemical LTD.) in order to investigate clearly so that the gel was rinsed and/or decolored by an evaporated water. A plate of the gel was recorded on the videotape to digitalize, analyze and illustrate. Criteria of position, contrast, radius and/or symmetry of precipitin line were used to judge a production of immunoglobulin. Generally, when amount of a newly produced antibody (or sample serum) is large, position of precipitin line is shifted toward the antibody which has been replaced in a groove and contrast of precipitin line is reduced. When a amount of a newly produced antibody is less, position of precipitin line is nearer to an axis of the sample hole and contrast of precipitin line toward the sample hole is resuced. Furthermore, when a molecular weight is smaller, position of precipitin line is shifted toward the antibody which has been replaced in a groove and radius of precipitin line becomes larger. In contrast, when a molecular weight is larger, a radius of precipitin line becomes smaller. When a newly produced antibody is homogenous, precipitin line is symmetrical. And, when a newly produced antibody is heterogeneous, precipitin line is asymmetrical.

Mouse melanoma cells (B16; purchased from Riken Cell Bank) was incubated with 2 ml of culture medium (Minimum Essential Medium, Gibco Co.) including bovine fetus serum (Dai-Nipon Pharmace. Co.) at a temperature of 37 °C and 5% CO₂ for 30 hours in the incubator. When a number of cell becomes to around 2x10⁵ , cell death is induced by an addition of 4 µl of Yoshixol which concentration is 2M with ethanol. Then, the culture medium which contains extincted cells was centrifuged at 3,000 cycles/min for 5 minutes so that it's supemant was removed. After adding physiological saline of 0.9 cc into the sedimented component and stirring, a centrifuging separation was performed at 1,000 cycle for 5 minutes. This procedure was repeated twice. Then, after adding physiological saline of 0.9 cc in the rinsed reside of sediment and stirring, the mixtured solution was filtered by miripore-filter of cellulose acetate with 0.45 micrometer pore. This filtered solution of 0.2 cc was injected intraperitoneally in C57/BL mice (female, 6 weeks, purchased from Nihon SLC Co.). Thirty days after the injection of the filtered solution, 0.3 cc of mice melanoma cells (2 x 10⁵/ml) which has been cultured was transplanted on a foot-pat of mice in which were injected or were not injected the filtered solution, so that local growth and metastasis to lung of melanoma cells B16 were investigated. Subsequently, as a result, a gain weight of distal leg from knee joint in the control mice was 120 mg on an average at 45 days after the cell transplantation. However, a gain weight of distal leg from knee joint in the treated mice with the filtered solution was 11 mg on an average at 45 days after the cell transplantation. In addition, on an pathological investigation of lung metastasis, a pine-point like metastasis on the lung was more than 122 on an average in the control group and was less than 10 in the treated group. This result shows that growth and metastasis of melanoma is blocked by promoting a new production of antibody in mice, resulting from using substances of extincted cell by Yoshixol is used as an antigen.

Morphological changes appeared after treatment with Yoshixol on mice melanoma cells B16 were investigated by scanning electron microscopy (Nihon Densi Co. JSM-6000F) as same as leukemic cells. In non-treated group, a aspect of cultured cells is proliferative pattern likely to regular rocky wall, an existence of mitotic cells and a normal intracellular appearance. An intercellular matrix is filled with extracellular substances (Figure 5a). In contrast, morphological aspects of cultured cells in treated with 4 ul of Yoshixol showed various irregular appearances such as destroyed structure of cellular membranes and/or organelle, and showed a separation of connection between cells and of various sizes of particles consisted of cellular membranes from cells (Figure 5b). And, cultured cells in treated group showed various patterns of destruction and did not show an adhesion and/or aggregation between cells, which indicate a similar morphological aspect to natural cell death or apoptosis (Figure 5c). These particles fragmented cellular components (10-100 nm), namely proteoglycans, act on an antigen resulting in phagocytotic effects of macrophages and/or lymphocytes. So that, those particles are combined into physiological immune surveillance mechanism in vivo. Subsequently, these morphological aspects suggest that antibody to recognize melanoma cell B16 in vivo is produced, resulting in molecular recognition.

Mouse melanoma cells (B16; purchased from Riken Cell Bank) was incubated with 2 ml of culture medium (Minimum Essential Medium, Gibco Co.) including bovine fetus serum (Dai-Nipon Pharmace. Co.) at a temperature of 37 °C and 5% CO₂ for 30 hours in the incubator. When a number of cell becomes to around 2x10⁵ , cell death is induced by an addition of 4 µl of Yoshixol which concentration is 2M with ethanol After confirming extinction of cells in culture medium, the culture medium which contains extincted cells was centrifuged at 3,000 cycles/min for 5 minutes so that it's supemant was removed. After adding physiological saline of 0.9 cc into the sedimented component and stirring, a centrifuging separation was performed at 1,000 cycle for 5 minutes. This procedure was repeated twice. Then, after adding physiological saline of 0.9 cc in the rinsed reside of sediment and stirring, the mixtured solution was filtered by miripore-filter of cellulose acetate with 0.45 micrometer pore. This filtered solution of 1 cc was injected intravenously in a rabbit. One month after the injection, the rabbit was sacrificed by bleeding under Nembutal anesthesia so that the serum was obtained after removal of cell components by a centrifugation. This serum was examined by an agar-gel diffusion of rabbit serum antibody against goat serum (Jackson ImmunoResearch Lab, PA, USA) in order to define whether this serum has been produced an antibody (immunoglobulin) against mice leukemic cells. Subsequently, it was confirmed to be formed a new band of immunoglobulin as shown in Figure 6.

A mechanism of effects and actions in this invention was explained by using Yoshixol as a representative example of Depressant of Functions developed by Molecule which has been disclosed in International Publication No. WO96/07403.

### <Summary of primary effect and mechanism, and it's significance>

In this invention, a wide-ranged and concrete effects and/or action concerning on molecular recognition, generation of function, signal transduction and antigen-antibody reaction was discussed briefly. However, to cite each reference about each scientific back-ground is far from the aim of this application so that the following two issues are cited as references of scientifically known events. < ref. 1> by Bern and Levy, Physiology, Sanders Publishing Inc.: < ref. 2> by Alberts, Ray, Lewis, Raff, Roberts and Watson, The Molecular Biology of the CELL, Garland Publishing Inc.:

In an immune system, it is divided into following two pathways. In the humoral antibody response as the first pathway, a produced antibody which circulates into the blood stream causes a specific ligand with an antigen of foreign body which induced a production of the antibody. When an antibody binds with the antigen, phagocyte becomes to easily capture the antigen and, proteins which are called as complements in blood become to be activated by antibody, resulting in a promotion of destroying the antigen. Cell-mediated immune response as the second pathway is a response to produce specific cells which is able to react with a foreign body antigen on the surface of host cells. These cells act against an antigen, resulting from killing host cells when antigen is an infectious virus and from inducing another cells such as phagocytes with a potency of destroying antigen. Moreover, it is a fundamental knowledge that T cell relates to cellular immune response and B cell produce an antibody. B cell produces an antibody-molecule with antigen-binding site which is different between each clone. Firstly, cells bind antibody-molecule to the cellular membrane which acts as surface receptor of the cells against an antigen. It is known that an antigen links to this binding site so that B cells are activated, resulting in a proliferation of B cells. The cells become to produce a lot of antibody, resulting in a release into the blood circulation.

From the fact which a three-dimensional structure of protein molecule such as antibody is determined by only an amino acid sequence, it recovers to be able to bind with an antigen although a denaturated (unfolding) antibody-molecule has not an antigenic potency. When antigen links with this surface receptor of cells, cells are activated resulting in a proliferation and growth. These foreign body antigens have been already provided the receptors against a homologous specificity of the antigen, resulting in specific stimulation of cells which are into a reactive stage. This is the reason why antigenic specificity appears in an immune response and is a fundamental basis of an immune surveillance mechanism. Moreover, it is thought that most macromolecules including all of proteins and polysaccharides have a potency to become antigen.

Antigen determinant is a binding site with antigen-binding site of receptor of antibody-molecule or lymphocytes, which exists on the surface of antigen. Hapten is a molecule which is not able to be induce an immune response and is able to produce a specific ligand with receptor of antibody or lymphocytes. Hapten becomes a antigen when it is linked with an carrier of adequate macromolecules. Hapten which have been used in immunological experiment is dinitrophenol (DNP). Generally, DNP is used as an antigen by binding with proteins.

Any types of antibodies have two characters; one is a membrane-binding type which acts as a specific surface receptor of cells against antigen and another is to exist as a water soluble secretary type. IgM and IgD are major parts of antibody on the surface of B cells which are in a resting stage.

A binding between antigen and antibody is reversible as a binding between substrate and enzyme. This binding is a summation of many non-covalent binds with relatively week force including in hydrophobic bind, hydrogen bind, van der Waals force and ion interaction. These week forces act efficiently when a position of atoms within antigen-molecule is fitted to the homologous fold on the surface of antibody resulting from sufficiently closing to antibody (It has been understood as a relationship between key and keyhole).

Moreover, antiserum produced against a kind of antigen generally creates a crosslink with antigen because that antiserum is a complex materials with various antibodies which are able to react with various antigen determinants. If an combination of antigen and antibody is an adequate binding affinity and a formation of large aggregation, a size of antigen-antibody complex is determined by a relative molar concentration of antigen and antibody. Producing and/or manufacturing processes of vaccines and antibodies are reasonable and appropriate on a basis of chemical property of Yoshixol which was used as a representative molecule in examples of this invention.

The successful result of immunochemistry at the present time is that an idiotype of antibody is a fundamental basis of immune network. Antibody itself plays a important role of control of immune-response as well as defense of organism against infections. Thus, when antibody is released against an antigen and is linked with the antigen, a receptor on B cells becomes to be stimulated because of loss of binding with antigen. So that, an immune response is stopped. This system is a simple feed-back inhibitory mechanism. In addition, antibody consists of a part of immunological network and plays more complicated roles on immunological modulation. This system is an acquired defense and/or survival mechanism of organism. On the basis of this network system, specificity and/or diversity of species is preserved. Also, a significance of this invention is to utilize a diversity on this evolutionary event.

In addition, antibody itself has an antigenic property so that it is possible to produce an antibody which is recognized a part of C.V region of immunoglobulin chain as a antigen determinant. When antigen-binding site becomes to be such antigen determinant namely on the V region of L.H chain, this antibody is termed as an idiotype antibody. A different antigen-binding site becomes to be a different idiotype antibody so that more than million of antigen-binding site with animals becomes to be more than million of idiotype antibody. Because of a less existence of each idiotype antibody in body, animals do not to become to an immune tolerance. In contrast, a response to both T cells and B cells occurred easily.

For example, it is thought that animals which have been immunized by antigen A produces much amount of anti-A antibody so that animals produce antibody against idiotype antibody of anti-A antibody. Following this process, animals produce antibody against anti-idiotype antibody. This reaction continues step wisely likely to a chain reaction. An existence of this immune network mechanism has been proofed by a fact that a major part of antibody produced by an initial response to antigen becomes a same idiotype antibody. Such simple immune response causes to produce antibody which has a property of specific recognition against idiotype antibody and to activate T cells. Subsequently, an activation of lymphocytes the idiotype antibody as receptors becomes to be inhibited or promoted.

There are many biological significances which individuals themselves are able to produce own needed antibody against idiotype antibody. Probably, there are a number of idiotype antibodies at least as same as a kinds of antigen determinants. In other wards, a standard antigen determinant has to recognize one of idiotype antibody at least in own immune system. An antigen determinant of these immune system forms latently a complex network of overall interaction between idiotype antibody and anti-idiotype antibody. In addition, it seems that T cells and B cells hold a little of idiotype antibody in common at least so that both types of lymphocytes are probably related to these kinds of networks. Therefore, it has been recently suggested a concept that immune responses are reflectable vibrations (likely to attenuating oscillation) better than these responses are individual response to antigen on lymphocytes. This possible immunological concept is identical to the fundamental idea of this invention.

A major component of chain reaction of protein degradation on such immune response is C3 component of complement so that an activation due to such degradation becomes to be a central reaction of a serial reaction of activating complement. However, a kind of polysaccharides which exist on a cellular surface of microorganisms preserves C3b-like molecule with such membrane-binding property and avoids to degenerate and/or destroy these substances. It has been known that on chain reaction of complements, a small fragment of proteins with a biological activity is produced by degradation of various components of proteins. It has been thought that chain reaction of this complement is controlled with an extreme planning to attack only a near-closed membrane. A loss of these active components is occurred at least by two processes. Firstly, when a specific blocking protein in blood is activated by a protein degradation, an active component plays a role of an ending function of chain reaction by a biding and/or fragmentation of a special component of protein. For example, a blocking protein prevents a promoting of a further action by a ligand formation with an activated component of C1 complexes. Furthermore, other blocking proteins exist in blood. These proteins cause a lack of activation by fragmenting Cab. It is reasonable from a fact that if these blocking factors do not exist, C3 complement has to be completely exhausted by an action of positive feed-back of alternative pathway. Secondly, an activated component in chain reaction has an unstable component so that this component plays an important role on control of the reaction. These unstable components become rapidly to loss a action when these do not bind with an adequate component on chain reaction or with a near-closed membrane, namely concerning on C4 and C3b. Either component becomes an activated one with a short life-span resulting from a serial and rapid change in conformation when the component is fragmented. This activated one has a hydrophobic region and a highly reactive side chain of glutamic acid, resulting from a mechanical fragmentation of a specific thioeter ligand in proteins. Subsequently, this glutamic acid immediately forms a covalent binding with proteins or polysaccharides of a near-closed membrane. A half time of this activated one is extremely short (less than 0.1 msec). Therefore, C4 and/or C3b is not able to bind only to a near-closed membrane with a necessary complement for activation. Thus, it seems that an attack of complement is limited to the surface of microorganism and is not expanded to normal host cells existed closely. In addition, a fragmentation of thioester between side chains of proteins generates to produce an extreme reactive carbonyl substrate resulting in covalent binding with macromolecule so that ester or amido linkages are formed. However, theses abilities of proteins disappears within 60 microseconds of a half life so that an reactive acceptor site is limited in a membrane closely to fragmented and/or activated site of peptide binding.

An antigen idiotype antibody which causes chain reaction of protein degradation of complement binds not only with anti-X antibody but with B cells which has an identical surface antibody as a receptor against antigen X. When anti idiotype antibody binds with a receptor on the surface of B cells, ability of recognition and/or response on B cells against antigen X is disturbed. It has been suggested a possibility that common idiotype antibody concerning to receptors of both B and T cells is corded by a gene fragment which determines V region of H chain on immunoglobulin. However, another possibility is that a receptor ofT cells is produced by a gene fragment which cords VH region of general antibody because of a blend new class of immunoglobulin. It has been suggested that B cells and T cells which react with same antigen become to induce an identical idiotype antibody (antigen determinant which exits on the binding site of antibody) on a surface receptor of the cells.

When cytotoxic T cells contact to cells of different species and/or mammalian cells infected by virus, those cytotoxic T cells become to be effector cells which are activated for a few days so that those T cells kill the cells of different species and/or mammalian cells by a specific linkage with a triggered cell of the activation. Helper T cells are needed to produce antibody responded to antigen, however, there are many antigens which are able to activate B cells without assistance of T cells. Such T cell independent antigen is a large polymer generally with a repeated structure of same antigen determinant. A recognition of a control T lymphocyte to a targeted cell is an interaction between idiotype antibody and anti-idiotype antibody as a one or reaction pathways. Two types of T cells are found on a response to antibody, which response is constituted almostly by identical idiotype antibodies. First one is to recognize a foreign body antigen and second is an idiotype antibody molecule of membrane binding which acts on the surface of B cells as a receptor.

The following comments is pointed out although those are repeatedly indicated; idiotype vaccine has been researched and developed as an ideal one. This concept is that antibody molecule, but not other antibody, has an own specific molecular structure which is able to be fitted with an antigen determinant so that an immunization of the antibody to animals produces antibody against it's determinant site. This antibody is anti-idiotype antibody. Therefore, an excellent linkage occurs between anti-idiotype antibody and antigen binding site (idiotype) of an original antibody. These indicate an existence of common structure between antigen determinant and anti-idiotype antibody. Thus, anti-idiotype antibody has a possibility to be applied as another antigen. This mention does not indicate to rule out the combinated manipulation and/or preparation, when it is needed, of conventional methods such as inactivation and/or live attenuation. These anti-idiotype antibody is a biohazard-less for a handling because of antibody molecule and is able to induce a species specificity selectively and combinationaly. Thus, DNA recombination has a possibility to mislead a part of saccharide chain instead of adequate part of peptide region. The idiotype vaccine and/or anti-idiotype vaccine has a characteristic property to be a antibody more closed to be needed.

As one of immunological surveillance system, tumor-related antigen is to induce immune response to tumor cells. A temporal existence of this tumor-related antigen appears at a initial stage of development so that this antigen is disappeared in normal development. However, there are several types of abnormal appearances such as α -fetoprotein which is appeared again after the disappearance by a formation of tumor, antigen which is appeared by a formation of tumor originated another tissue from an initial tumor tissue, antigen which allogenic antigen is induced by a formation of tumor and which is not preexisted in individual, antigen originated by virus, antigen which is induced by activation of oncogenes, antigen which is produced a new antigen with a changed saccharide chain of cellular membrane substances by a abnormal metabolism associated with a formation of tumor and idiotype antigen of surface immunoglobulin in a tumor ofB cell groups. In those examples, antigen which is appeared only in tumor cells, but not in any normal cells, is called as tumor related antigen. However, a little of existences has been identified at the present time. Thus, an energetic research activities has been focused at a level of genetic analysis. This invention is used antitumor or anticancer action which has been disclosured internationally in the international patent file (PTC: WO96/07403) related on inhibitory or blocking agents of molecular generating and/or inducing functions, to induce a specificity of tumor cells. Also, this invention is expanded and/or utilized a mechanism of action which is able to induce a destroyed microstructure dependent on morphological and functional specificity of tumor cells and to induce an apoptosis-like cell death. It is as far as possible to induce a specific antibody with a complexities against original tumor cells, resulting from an application of this extreme specific and fragmented complex substance as an antigen. In addition, this antibody is applied as a antigen to produce a more highly sensitive and/or specific antibody.

Furthermore, a trial of tumor therapy has been performed by utilizing antitumor immune response. A substance to promote biological reaction against tumor is called as a biological response modifier. Such substances are bacterial-originated substances, fungal-originated substances, antibiotics and cytokines such as interleukin 2, interferon and tumor necrosing factor). Various examinations have been performed. In addition, an antibody which is produced by utilizing idiotype antibody and/or anti-idiotyped antibody as antigen, which is also produced by antigenic substance inducer mentioned in this invention, is used to vital tissues obtained by endoscopic manipulation as well as surgical excision, removal and biopsy, utilizing with tissue culture. Thus, it is possible to induce an extreme specific anti-tumor effect depending on individual patient and/or various differences between oncogenic pathogenesis against several problems such as tissue specificity and tumor specificity of patients themselves. This possibility has been suggested by use of animals in same species. Tumor cells which occurred in an animal are killed by an adequate manipulation so that the killed cells is injected into an another normal animal. When alive tumor cells are implanted in non-treated animal, the tumor cells grow and proliferate so that the animal was dead by tumor. However, an investigation shows that in the pretreated animal, tumor does not grow and is rejected. This fact is extremely similar to prevention of onset of infectious diseases by vaccination. However, an adequate method of killing cells with representative examples and reproducibility has not been presented. A fundamental logic in this invention is identical to that mentioned in the international patent file (PTC: WO96/07403) related on inhibitory or blocking agents of molecular generating and/or inducing functions, in relationship of same events integrating between inside and outside. This invention is to disclosure a bio-medical logical process with representative examples such as anti-tumor and antineoplastic action. In addition, a planning manipulation of an specific activated immune response to prevent and/or treat diseases becomes to be possible and has been expected. Vaccine precursor or vaccine which is produced by use of antigenic substance inducer in this invention, has been expected as a beneficial one.

Moreover, vaccine precursor or vaccine, which is produced by use of antigenic substance inducer in this invention, are able to control a molecular recognition selectively and/or specifically.

A major significance of this invention is firstly demonstrated to obtain an antigenic substance which has a fundamental structure close to species specificity, resulting from quantum thermodynamic changes in function and/or blocking action of biological functions which is generated and/or induced by multidimensional structure of macromolecules. Because various morphological aspects and functions of organism and non-organism are induced and/or generated by multidimensional structures of macromolecules. As additional significance, this invention is demonstrated firstly to produce an antibody with a molecular recognition against substances with a high diversity in vivo. Moreover, industrial significance as well as medical significance demonstrated in this invention is also existed to resolve unknown signal transduction system and/or processing system of complexed biological immune surveillance network such as cording, receiving and decoding.

Here, a fundamental principle of this invention is summarized. The purpose of this invention is to be acquired a ability of antibody recognition in organism and to produce and/or manufacture an antitumor agent vaccine using a fragmented structural substance as an antigen, resulting from extinction of obtained by tumor cells. Those effects are utilized a destroying and extincting action of Yoshixol on organism and/or a decomposition action and fragment formation of Yoshixol to become non-functional macromolecules of constituted components of cellular structure. These points are suggested by the following effects of Yoshixol which is a representative one of inhibitory or blocking agents of molecular generating and/or inducing functions. These effects is 1) that Yoshixol is able to destroy cells (both of eukaryotes and prokaryotes) into granular particles with a size of 10-400 nm, 2) that there is a difference between morphological aspects of fragmented particles depending on species, 3) that a fragmented structure is easily deformed and solved by a heat accumulation of electron beam and is similar to morphological aspects of peptidoglycans and proteoglycans, 5) that peptidoglycans and proteoglycans play an important role of mophogenetic maintenance of cells, are different between species and induce a species specificity, 5) peptidoglycans and proteoglycans play an important role on cytokines, surface antibodies, functional inductions, structural formations and signal transduction (high affinity receptors), moreover 6) that peptidoglycans and proteoglycans are major substances of extracellular matrix such as condroitin, fibrin and collagen, 7) that Yoshixol is able to induce apoptosis, 8) Yoshixol affects on methylation of G-proteins, a metylating stage, RNA-protein synthesizing stage of cells because of a fact that a flowcytometric analysis on cell cycle and action stage of Yoshixol has suggested an arrest at Go/Gl - early S phase, and 9) that Yoshixol has a mechanism according to electron density and electron orbital (quantum theory) resulting in changes in multidimensional conformation of complexed proteins such as enzymes ( for examples, lipase, HIV protease and fanesyl transferase) and G-proteins which are known as signal interfaced substances so that Yoshixol inhibits and/or control functional inductions.

Therefore, Yoshixol is able to cause a fragmentation of morphological structure and to arrest functions with a preserving process as far as possible of morphological elements and functions with a diversity of cells depending on DNA. Therefore, a substance (without heating and chemical manipulation) is obtained depending on each specificity with cells. Animals which are received such fragmented complex macromolecules as an antigen produce antibody themselves against the received complex macromolecular substance so that antibody becomes to adequately and correctly recognize original cells resulting in removal of invasive non-self cells as foreign body. Thus, this mechanism is to selectively generate self-defense mechanism of antigen-antibody reaction and is to establish a new immune surveillance system. In order to understand proliferation, mitosis, transport of substance and signal transduction between each cell and to utilize these scientific facts, however, it is needed to accumulate further scientific knowledges at the present. Thus, this invention is able to provide vaccine precursor and vaccine, which is produced by use of combined substances as antigen at a stage of functional induction or at dynamic (for examples, chemical reaction, physical reaction and it's interaction) activating stage of cells.

Thus, a treatment with Yoshixol is able to obtain an action of molecular precognition which induces superselectiveness and/or specificity. Furthermore, a molecular weight of Yoshixol used in this invention is extremely small so that it has a property of hapten and of direct actions on tumor cells, various types of pathologic organs and tissues. In addition, a treatment with Yoshixol promotes an additional activation of immune surveillance system when it is applied to obtain a direct action. Thus, it is easily thought with a reasonability to have a possibility of accumulating a synergistic therapeutic efficiency.

## Claims

1. Use of a vaccine or a vaccine precursor obtained by in vitro treating a pathogen with a compound of 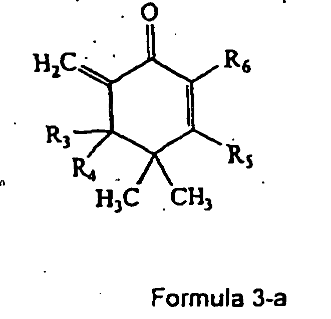 for the manufacture of an antitumor agent,
wherein in Formula 3-a
(i) R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group;
(ii) one or more of R3 and R4, and/or one or more of R5 and R6 may be substituted or non-substituted cyclopentyl group; substituted or non-substituted cyclohexyl group; or substituted or non-substituted naphthyl group;
(iii) R5 and R6 may form a ring by binding with another condensation polycyclic hydrocarbon compound or heterocyclic compound;
(iv) one or more of R3, R4, R5 and R6 may be substituted by one or more of substituents selected from the group consisting of halogen atom, cyano group, protected or non-protected carboxyl group, protected or non-protected hydroxyl group protected or non-protected amino group, C1-C6 alkyl group, C1-C6 alkoxy group, C1-C7 alkoxy carbonyl group, aryl group, C3-C6 cycloalkyl group, C1-C6 acylamino group, C1-C6 acyloxy group, C2-C6 alkenyl group, C1-C6 trihalogenoalkyl group, C1-C6 alkylamino group, and C1-C6 dialkylamino group:
(v) R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C1-C6 alkyl group, protected or non-protected hydroxyalkyl group, and C3-C6 cycloalkylamino group;
(vi) when one or more of R3, R4, R5 and R6 are alkyl groups, terminal end(s) of the alkyl group(s) may be substituted by C3-C8 cycloalkyl group, and
wherein the pathogen is tumor cells.

2. Use of a vaccine or a vaccine procursor obtained by in vitro treating cells of pathological tissues with a compound of 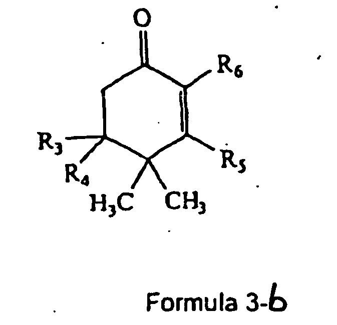 for the manufacture of on antitumor agent, wherein in Formula 3-b
(i) R3, R4, R5 and R6 represent independently hydrogen atom; halogen atom; C1-C6 alkyl group; amidino group; C3-C8 cycloalkyl group; C1-C6 alkoxy C1-C6 alkyl group; aryl group; allyl group; aralkyl group in which one or more C1-C6 alkyl groups are bound to an aromatic ring selected from the group consisting of benzene, naphthalene and anthracene ring; C1-C6 alkylene group; benzoyl group; cinnamyl group; cinnamoyl group or furoyl group;
(ii) one or more of R3 and R4, and/or one or more of R5 and R6 may be substituted or non-substituted cyclopentyl group; substituted or non-substituted cyclohexyl group; or substituted or non-substituted naphthyl group;
(iii) R5 and R6 may form a ring by binding with another condensation polycyclic hydrocarbon compound or heterocyclic compound;
(iv) one or more of R3, R4, R5 and R6 may be substituted by one or more of substituents selected from the group consisting of halogen atom, cyano group, protected or non-protected carboxyl group, protected or non-protected hydroxyl group, protected or non-protected amino group, C1-C6 alkyl group, C1-C6 alkoxy group, C1-C7 alkoxy carbonyl group, aryl group, C3-C6 cycloalkyl group, C1-C6 acylamino group, C1-C6 acyloxy group, C2-C6 alkenyl group, C1-C6 trihalogenoalkyl group, C1-C6 alkylamino group, and C1-C6 dialkylamino group;
(v) R5 may be substituted by one or more substituents selected from the group consisting of halogen atom, C1-C6 alkyl group, protected or non-protected carboxyl group, protected or non-protected hydroxyl group, protected or non-protected amino group, protected or non-protected C1-C6 alkylamino group, protected or non-protected C1-C6 aminoalkyl group, protected or non-protected C1-C6 alkylamino C 1-C6 alkyl group, protected or non-protected hydroxyalkyl group, and C3-C6 cycloalkylamino group;
(vi) when one or more of R3, R4, R5 and R6 are alkyl groups, terminal end(s) of the alkyl group(s) may be substituted by C3-C8 cycloalkyl group, and wherein the cells of pathological tissues are tumor cells.

3. Use according to claim 1 or 2, wherein in Formula 3-a or 3-b, respectively, said aryl group in (i) and (iv) is phenyl, tollyl, xylyl or naphthyl group; said substituted cyclopentyl group in (ii) is cyclopentylamino group or cyclopentylcarbinol group, said substituted cyclohexyl group in (ii) is cyclohexylamino group, cyclohexylaldehyde group or cyclohexyl acetic acid group, and said substituted naphthyl group in (ii) is naphtylamino group or naphthylamino sulfonic acid group; and
said condensation polycyclic hydrocarbon compound in (iii) is pentalene, indene, naphthalene, azulene, heptalene, biphenylene, indacene, acenaphthylene, fluorene, phenalene, phenanthrene, anthracene, pentacene, hexacene, dibenzophenanthrene, 1H-cyclopentacyclooctene or benzocyclooctene, and said heterocyclic compound is furan, thiophene, pyrrole, γ-pyran, γ-thipyran, pyridine, thiazole, imidazole pyrimidine, indole or quinoline.

4. Use according to claim 1 or 2, wherein in Formula 3-a or 3-b, respectively, R3, R4, R5 and R6 represent hydrogen atoms.

## Patentansprüche

1. Verwendung eines Impfstoffs oder Impfstoffvorläufers, erhalten durch in vitro Behandlung eines Pathogens mit einer Verbindung der Formel 3-a 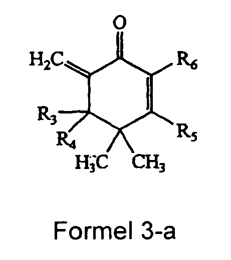 zur Herstellung eines Antitumormittels,
worin in Formel 3-a
(i) R3, R4, R5 und R6 unabhängig ein Wasserstoffatom, ein Halogenatom, eine C1-C6-Alkylgruppe, eine Amidinogruppe, eine C3-C8-Cycloalkylgruppe, eine C1-C6-Alkoxy-C1-C6-alkylgruppe, eine Arylgruppe, eine Allylgruppe, eine Aralkylgruppe, worin eine oder mehrere C1-C6-Alkylgruppen an einen aromatischen Ring, ausgewählt aus der Gruppe bestehend aus Benzol-, Naphthalin- und Antharcenring, gebunden sind, eine C1-C6-Alkylengruppe, eine Benzoylgruppe, eine Cinnamylgruppe, eine Cinnamoylgruppe oder eine Furoylgruppe darstellen;
(ii) eines oder mehrere von R3 und R4 und/oder eines oder mehrere von R5 und R6 eine substituierte oder unsubstituierte Cyclopentylgruppe, eine substituierte oder unsubstituierte Cyclohexylgruppe oder eine substituierte oder unsubstituierte Naphthylgruppe ist/sind;
(iii) R5 und R6 durch Bindung an eine andere kondensierte polycyclische Kohlenwasserstoffverbindung oder heterocyclische Verbindung einen Ring bilden können;
(iv) eines oder mehrere von R3, R4, R5 und R6 mit einem oder mehreren Substituenten, ausgewählt aus der Gruppe bestehend aus einem Halogenatom, einer Cyanogruppe, einer geschützten oder ungeschützten Carboxylgruppe, einer geschützten oder ungeschützten Hydroxylgruppe, einer geschützten oder ungeschützten Aminogruppe, einer C1-C6-Alkylgruppe, einer C1-C6 Alkoxygruppe, einer C1-C7-Alkoxycarbonylgruppe, einer Arylgruppe, einer C3-C6-Cycloalkylgruppe, einer C1-C6-Acylaminogruppe, einer C1-C6-Acyloxygruppe, einer C2-C6-Alkenylgruppe, einer C1-C6-Trihalogenoalkylgruppe, einer C1-C6-Alkylaminogruppe und einer C1-C6-Dialkylaminogruppe, substituiert sein kann/können;
(v) R5 mit einem oder mehreren Substituenten, ausgewählt aus der Gruppe bestehend aus einem Halogenatom, einer C1-C6-Alkylgruppe, einer geschützten oder ungeschützten Carboxylgruppe, einer geschützten oder ungeschützten Hydroxylgruppe, einer geschützten oder ungeschützten Aminogruppe, einer geschützten oder ungeschützten C1-C6-Alkylaminogruppe, einer geschützten oder ungeschützten C1-C6-Aminoalkylgruppe, einer geschützten oder ungeschützten C1-C6-Alkylamino-C1-C6-alkylgruppe, einer geschützten oder ungeschützten Hydroxyalkylgruppe und einer C3-C6-Cycloalkylaminogruppe, substituiert sein kann;
(vi) das endständige Ende der Alkylgruppe(n) mit C3-C8-Cycloalkylgruppen substituiert sein kann, wenn eines oder mehrere von R3, R4, R5 und R6 Alkylgruppen sind,
und worin das Pathogen Tumorzellen ist.

2. Verwendung eines Impfstoffs oder eines Impfstoffvorläufers, erhalten durch in vitro Behandlung von Zellen aus pathologischem Gewebe mit einer Verbindung der Formel 3-b 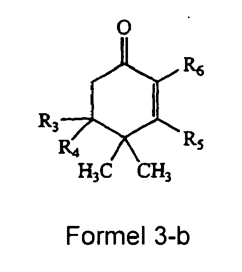 zur Herstellung eines Antitumormittels,
worin in Formel 3-b
(i) R3, R4, R5 und R6 unabhängig ein Wasserstoffatom, ein Halogenatom, eine C1-C6-Alkylgruppe, eine Amidinogruppe, eine C3-C8-Cycloalkylgruppe, eine C1-C6-Alkoxy-C1-C6-alkylgruppe, eine Arylgruppe, eine Allylgruppe, eine Aralkylgruppe, worin eine oder mehrere C1-C6-Alkylgruppen an einen aromatischen Ring, ausgewählt aus der Gruppe bestehend aus Benzol-, Naphthalin- und Antharcenring, gebunden sind, eine C1-C6-Alkylgruppe, eine Benzoylgruppe, eine Cinnamylgruppe, eine Cinnamoylgruppe oder eine Furoylgruppe darstellen;
(ii) eines oder mehrere von R3 und R4 und/oder eines oder mehrere von R5 und R6 eine substituierte oder unsubstituierte Cyclopentylgruppe, eine substituierte oder unsubstituierte Cyclohexylgruppe oder eine substituierte oder unsubstituierte Naphthylgruppe ist/sind;
(iii) R5 und R6 durch Bindung an eine andere kondensierte polycyclische Kohlenwasserstoffverbindung oder heterocyclische Verbindung einen Ring bilden können;
(iv) eines oder mehrere von R3, R4, R5 und R6 mit einem oder mehreren Substituenten, ausgewählt aus der Gruppe bestehend aus einem Halogenatom, einer Cyanogruppe, einer geschützten oder ungeschützten Carboxylgruppe, einer geschützten oder ungeschützten Hydroxylgruppe, einer geschützten oder ungeschützten Aminogruppe, einer C1-C6-Alkylgruppe, einer C1-C6 Alkoxygruppe, einer C1-C7-Alkoxycarbonylgruppe, einer Arylgruppe, einer C3-C6-Cycloalkylgruppe, einer C1-C6-Acylaminogruppe, einer C1-C6-Acyloxygruppe, einer C2-C6-Alkenylgruppe, einer C1-C6-Trihalogenoalkylgruppe, einer C1-C6-Alkylaminogruppe und einer C1-C6-Dialkylaminogruppe, substituiert sein kann/können;
(v) R5 mit einem oder mehreren Substituenten, ausgewählt aus der Gruppe bestehend aus einem Halogenatom, einer Cl-C6-Alkylgruppe, einer geschützten oder ungeschützten Carboxylgruppe, einer geschützten oder ungeschützten Hydroxylgruppe, einer geschützten oder ungeschützten Aminogruppe, einer geschützten oder ungeschützten C1-C6-Alkylaminogruppe, einer geschützten oder ungeschützten C1-C6-Aminoalkylgruppe, einer geschützten oder ungeschützten C1-C6-Alkylamino-C1-C6-alkylgruppe, einer geschützten oder ungeschützten Hydroxyalkylgruppe und einer C3-C6-Cycloalkylaminogruppe, substituiert sein kann;
(vi) das endständige Ende der Alkylgruppe(n) mit C3-C8-Cycloalkylgruppen substituiert sein kann, wenn eines oder mehrere von R3, R4, R5 und R6 Alkylgruppen sind,
und worin die Zellen des pathologischen Gewebes Tumorzellen sind.

3. Verwendung nach Anspruch 1 oder 2, worin in Formel 3-a bzw. 3-b die Arylgruppe in (i) und (iv) Phenyl, Tolyl, Xylyl oder Naphthyl ist, die substituierte Cyclopentylgruppe in (ii) Cyclopentylamino oder Cyclopentylcarbinol ist, die substituierte Cyclohexylgruppe in (ii) Cyclohexylamino, Cyclohexylaldehyd oder Cyclohexylessigsäure ist, und die substituierte Naphthylgruppe in (ii) Naphthylamino oder Naphthylaminosulfonsäure ist; und
die kondensierte polycyclische Kohlenwasserstoffgruppe in (iii) Pentalen, Inden, Naphthalin, Azulen, Heptalen, Biphenylen, Indacen, Acenaphthylen, Fluoren, Phenalen, Phenanthren, Anthracen, Pentacen, Hexacen, Dibenzophenanthren, 1 H-Cyclopentacycloocten oder Benzocycloocten ist, und die heterocyclische Verbindung Furan, Thiophen, Pyrrol, γ-Pyran, γ-Thipyran, Pyridin, Thiazol, Imidazolpyrimidin, Indol oder Chinolin ist.

4. Verwendung nach Anspruch 1 oder 2, worin in Formel 3-a bzw. 3-b R3, R4, R5 und R6 Wasserstoffatome darstellen.

## Revendications

1. Utilisation d'un vaccin ou d'un précurseur de vaccin obtenu par traitement *in vitro* d'un agent pathogène avec un composé de 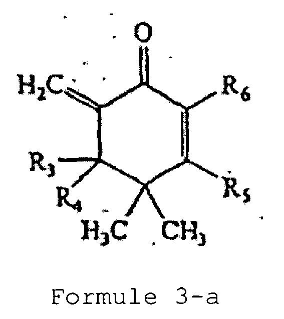 pour la fabrication d'un agent antitumoral,
où dans la formule 3-a
(i) R3, R4, R5 et R6 représentent indépendamment un atome d'hydrogène ; un atome d'halogène ; un groupe alkyle en C₁ à C₆ ; un groupe amidino ; un groupe cycloalkyle en C₃ à C₈ un groupe alcoxy en C₁ à C₆-alkyle en C₁ à C₆ ; un groupe aryle ; un groupe allyle ; un groupe aralkyle dans lequel un ou plusieurs groupes alkyle en C₁ à C₆ sont liés à un cycle aromatique choisi dans le groupe constitué des cycles benzène, naphtalène et anthracène ; un groupe alkylène en C₁ à C₆ ; un groupe benzoyle ; un groupe cinnamyle ; un groupe cinnamoyle ou un groupe furoyle ;
(ii) un ou plusieurs de R3 et R4, et/ou un ou plusieurs de R5 et R6 peuvent représenter un groupe cyclopentyle substitué ou non substitué ; un groupe cyclohexyle substitué ou non substitué ; ou un groupe naphtyle substitué ou non substitué ;
(iii) R5 et R6 peuvent former un cycle par liaison avec un autre composé hydrocarbure polycyclique de condensation ou un composé hétérocyclique ;
(iv) un ou plusieurs de R3, R4, R5 et R6 peuvent être substitués par un ou plusieurs de substituants choisis dans le groupe constitué par un atome d'halogène, un groupe cyano, un groupe carboxyle protégé ou non protégé, un groupe hydroxyle protégé ou non protégé, un groupe amino protégé ou non protégé, un groupe alkyle en C₁ à C₆, un groupe alcoxy en C₁ à C₆, un groupe alcoxy en C₁ à C₆-carbonyle, un groupe aryle, un groupe cycloalkyle en C₃ à C₆, un groupe acyl en C₁ à C₆-amino, un groupe acyl en C₁ à C₆-oxy, un groupe alcényle en C₂ à C₆, un groupe trihalogénoalkyle en C₁ à C₆, un groupe alkyl en C₁ à C₆-amino et un groupe dialkyl en C₁ à C₆-amino ;
(v) R5 peut être substitué par un ou plusieurs substituants choisis dans le groupe constitué par un atome d'halogène, un groupe alkyle en C₁ à C₆, un groupe carboxyle protégé ou non protégé, un groupe hydroxyle protégé ou non protégé, un groupe amino protégé ou non protégé, un groupe alkyl en C₁ à C₆-amino protégé ou non protégé, un groupe aminoalkyle en C₁ à C₆ protégé ou non protégé, un groupe alkyl en C₁ à C₆-aminoalkyle en C₁ à C₆ protégé ou non protégé, un groupe hydroxyalkyle protégé ou non protégé et un groupe cycloalkyl en C₃ à C₆-amino ;
(vi) lorsqu'un ou plusieurs de R3, R4, R5 et R6 représentent des groupes alkyle, l'extrémité/les extrémités terminale(s) du/des groupe(s) alkyle peut/peuvent être substituée(s) par un groupe cycloalkyle en C₃ à C₈, et
où l'agent pathogène correspond à des cellules tumorales.

2. Utilisation d'un vaccin ou d'un précurseur de vaccin obtenu par traitement de cellules de tissus pathologiques avec un composé de 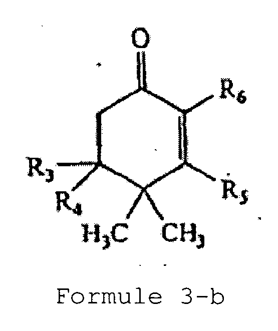 pour la fabrication d'un agent antitumoral,
où dans la formule 3-b
(i) R3, R4, R5 et R6 représentent indépendamment un atome d'hydrogène ; un atome d'halogène ; un groupe alkyle en C₁ à C₆ ; un groupe amidino ; un groupe cycloalkyle en C₃ à C₈ ; un groupe alcoxy en C₁ à C₆-alkyle en C₁ à C₆ ; un groupe aryle ; un groupe allyle ; un groupe aralkyle dans lequel un ou plusieurs groupes alkyle en C₁ à C₆ sont liés à un cycle aromatique choisi dans le groupe constitué des cycles benzène, naphtalène et anthracène ; un groupe alkylène en C₁ à C₆ ; un groupe benzoyle ; un groupe cinnamyle ; un groupe cinnamoyle ou un groupe furoyle ;
(ii) un ou plusieurs de R3 et R4, et/ou un ou plusieurs de R5 et R6 peuvent représenter un groupe cyclopentyle substitué ou non substitué ; un groupe cyclohexyle substitué ou non substitué ; ou un groupe naphtyle substitué ou non substitué ;
(iii) R5 et R6 peuvent former un cycle par liaison avec un autre composé hydrocarbure polycyclique de condensation ou un composé hétérocyclique ;
(iv) un ou plusieurs de R3, R4, R5 et R6 peuvent être substitués par un ou plusieurs de substituants choisis dans le groupe constitué par un atome d'halogène, un groupe cyano, un groupe carboxyle protégé ou non protégé, un groupe hydroxyle protégé ou non protégé, un groupe amino protégé ou non protégé, un groupe alkyle en C₁ à C₆, un groupe alcoxy en C₁ à C₆, un groupe alcoxy en C₁ à C₆-carbonyle, un groupe aryle, un groupe cycloalkyle en C₃ à C₆, un groupe acyl en C₁ à C₆-amino, un groupe acyl en C₁ à C₆-oxy, un groupe alcényle en C₂ à C₆, un groupe trihalogénoalkyle en C₁ à C₆, un groupe alkyl en C₁ à C₆-amino et un groupe dialkyl en C₁ à C₆-amino ;
(v) R5 peut être substitué par un ou plusieurs substituants choisis dans le groupe constitué par un atome d'halogène, un groupe alkyle en C₁ à C₆, un groupe carboxyle protégé ou non protégé, un groupe hydroxyle protégé ou non protégé, un groupe amino protégé ou non protégé, un groupe alkyl en C₁ à C₆-amino protégé ou non protégé, un groupe aminoalkyle en C₁ à C₆ protégé ou non protégé, un groupe alkyl en C₁ à C₆-aminoalkyle en C₁ à C₆ protégé ou non protégé, un groupe hydroxyalkyle protégé ou non protégé et un groupe cycloalkyl en C₃ à C₆-amino ;
(vi) lorsqu'un ou plusieurs de R3, R4, R5 et R6 représentent des groupes alkyle, l'extrémité/les extrémités terminale(s) du/des groupe(s) alkyle peut/peuvent être substituée(s) par un groupe cycloalkyle en C₃ à C₈, et
où les cellules des tissus pathologiques sont des cellules tumorales.

3. Utilisation selon la revendication 1 ou 2, où dans la formule 3-a ou 3-b, respectivement, ledit groupe aryle dans (i) et (iv) est un groupe phényle, tolyle, xylyle ou naphtyle ; ledit groupe cyclopentyle substitué dans (ii) est un groupe cyclopentylamino ou un groupe cyclopentylcarbinol, ledit groupe cyclohexyle substitué dans (ii) est un groupe cyclohexylamino, un groupe cyclohexylaldéhyde ou un groupe acide cyclohexyl-acétique, et ledit groupe naphtyle substitué dans (ii) est un groupe naphtylamino ou un groupe acide naphtylamino-sulfonique ; et
ledit composé hydrocarbure polycyclique de condensation dans (iii) est le pentalène, l'indène, le naphtalène, l'azulène, l'heptalène, le biphénylène, l'indacène, l'acénaphtylène, le fluorène, le phénalène, le phénanthrène, l'anthracène, le pentacène, l'hexacène, le dibenzophénanthrène, le 1H-cyclopentacyclooctène ou le benzocyclooctène, et ledit composé hétérocyclique est le furane, le thiophène, le pyrrole, le y-pyrane, le y-thiopyrane, la pyridine, le thiazole, l'imidazole, la pyrimidine, l'indole et la quinoléine.

4. Utilisation selon la revendication 1 ou 2, où dans la formule 3-a ou 3-b, respectivement, R3, R4, R5 et R6 représentent des atomes d'hydrogène.
